# EUROPEAN PATENT APPLICATION

(11) **EP 0 955 311 A2**
(43) Date of publication of application: **10.11.1999**
(21) Application number: 99107020.2
(22) Date of filing: 09.04.1999
(51) Int. Cl.: C07K 7/06, C07K 7/08, C07K 16/06, A61K 39/395, C07K 16/28

(54) **Peptide vaccine for canine allergy**

(30) Priority: 09.04.1998 US 58332; 30.03.1999 US 281761
(71) Applicant: IDEXX LABORATORIES, INC., Westbrook, ME 04092 (US)
(72) Inventor: Lawton, Robert, Gorham, Maine 04938 (US); Mermer, Brion, Cumberland, Maine 04092 (US); Francoeur, Greg, North Yarmouth, Maine 04097 (US)
(74) Representative: Viering, Jentschura & Partner

(57) **Abstract**

Disclosed is an isolated and purified peptide comprising a leucine positioned two peptide bonds away from a tyrosine-arginine pair, such as having the configuration: leucine-blank-blank-tyrosine-arginine, tyrosine-arginine-blank-blank-leucine, or, leucine-blank-blank-tyrosine-arginine-blank-blank-leucine, where blank is any amino acid. Other peptides of the invention include those with the sequence cysteine-blank-blank-proline-histidine-blank-blank-blank-cysteine; cysteine-blank-proline-histidine-blank-proline-blank-blank-cysteine. The peptides can be linked to a carrier such as a multiply antigen protein or a plant virus particle. Isolated and purified peptides of the invention can be used to induce canine autoantibodies directed to the canine IgE molecule.

## Description

This application is a continuation-in-part of copending application United States Serial Number 09/058,332, filed April 9, 1998, which is incorporated herein in its entirety.

### FIELD OF THE INVENTION

This invention concerns peptides. More particularly, the invention concerns compositions for administration to dogs, which actively provide immunity to the dog's immunoglobulin E molecules.

### BACKGROUND ART

It is estimated that up to 30% of all dogs suffer from allergies or allergy-related skin disorders. Specifically, allergic dermatitis has been estimated to affect between 3 and 15% of the entire canine population. Given the prevalence of allergies in dogs, there is a need to develop methods and compositions to properly diagnose and treat canine allergies.

The substances most likely to cause an allergic reaction vary from species to species. Common canine allergens include fleas, pollens, molds and dust. Allergy to fleas is believed to be the most common dog allergy. Typically, a flea's saliva is the allergen, and a single fleabite can cause substantial itching. An additional form of allergy in dogs is termed atopy. Atopy is a condition where a dog is allergic to inhalants such as pollens, molds or microscopic mites such as are found in house dust.

Antibody molecules play a role in allergic manifestations. In mammals, antibody molecules are classified into various isotypes referred to as IgA, IgD, IgE, IgG, and IgM. Antibody molecules consist of both heavy and light chain components. The heavy chains of molecules of a given isotype have extensive regions of amino acid sequence homology, and conversely have regions of difference from antibodies belonging to other isotypes. The shared regions of the heavy chains provide members of each isotype with common abilities to bind to certain cell surface receptors or to other macromolecules, such as complement. These heavy chain regions, therefore, serve to activate particular immune effector functions. Accordingly, separation of antibody molecules into isotypes serves to separate the antibodies according to a set of effector functions that they commonly activate.

In humans and dogs, immunoglobulin E (hereinafter IgE) is involved in allergy. Thus, IgE is the antibody type that is understood to be an important mediator of allergic responses, including Type I immediate hypersensitivity.

IgE molecules bind to mast cells and basophils. This binding occurs when the Fc region of the IgE molecule is bound to Fc receptors on the mast cells. When such bound IgE antibodies then bind to an allergen, the allergen cross-links multiple IgE antibodies on the cell surface. This cross-linking mediates Type I immediate hypersensitivity reactions and causes release of histamines and other molecules that produce symptoms associated with allergy.

Monoclonal antibodies having different degrees of sensitivity to canine IgE and IgG have been identified. (DeBoer, et al. Immunology and Immunopathology 37, 183-199 (1993).) DeBoer, et al. identified several monoclonal antibodies which had cross reactivity between IgG and IgE. (See, e.g., DeBoer, et al., Table 3 and accompanying text.) Three monoclonal antibodies (A5, D9, and B3) were identified by DeBoer et al., as having some affinity for canine IgE. Of the monoclonal antibodies identified in DeBoer et al., antibody D9 appeared to have the greatest degree of neutralization of Prausnitz-Kustner reactivity for atopic dog serum. In the context of canine allergy, DeBoer et al. proposed use of their monoclonal antibodies (MAbs) in the use of antigen-specific IgE ELISA, and for quantifying canine IgE. Additionally, they proposed use of their MAbs for immunostaining of Western Blot assays, to evaluate the molecular specificity of IgE antibodies, as well as for *in vitro* studies on degranulation of mast cells.

In humans the serum level of total IgE is diagnostic of allergic disease. To explore the possibility that the serum level of IgE might also be diagnostic of allergy in dogs, several studies were performed. (Hill and DeBoer Am. J. Yet. Res., (July 1994) *55(7)*, 944-48). Publications following the DeBoer article used a monoclonal antibody designated D9 in an ELISA assay having the following configuration: D9 was bound to a substrate, antibodies were captured by D9 and then D9 having a marker was used to flag the captured antibody. The Hill and DeBoer ELISA was used to establish the total amount of IgE in canine serum in an effort to diagnose canine allergy. In contrast to humans, the quantity of IgE determined to exist in canine circulation was of no use whatsoever in the diagnosis of allergy in dogs. (See, e.g., Abstract and Discussion Sections of Hill and DeBoer) This finding was in direct contrast to the situation in human immunology.

This divergent diagnostic result based on levels of IgE in humans compared to such levels in dogs, points out the difficulty of any attempt to correlate data between animals of two different genera. This difficulty is further exacerbated by the fact that dogs can be allergic to a different set of antigens than humans are. Fleas, for instance, are a severe problem for dogs, but not humans. Furthermore, in instances where dogs and humans appear to be allergic to the same allergen extract, studies by doctors Esch and Greer of Greer Laboratories, have indicated that the specific allergens in an allergen extract which produce canine disease are not necessarily the same allergens that produce disease in humans. For example, it is known that the immunodominant components of dust mite extracts are different in dogs than in humans.

The genomic sequences encoding human and murine IgE heavy chain constant region are known (For example, see Ishida et al., "The Nucleotide Sequence of the Mouse Immunoglobulin E Gene: Comparison with the Human Epsilon Gene Sequence", EMBO Journal *1*,1117-1123 (1982). A comparison of the human and murine genes shows that they possess 60% homology within exons, and 45-50% homology within introns, with various insertions and deletions.

Patel et al. published the nucleotide and predicted amino acid sequence for exons 1-4 of the heavy chain constant region of canine IgE in the article entitled "Sequence of the Dog Immunoglobulin Alpha and Epsilon Constant Region Genes," Immunogenetics *41*, 282-286 (March 22, 1995). The complete sequence of the canine IgE heavy chain constant region, with membrane bound portions encoded by exons 5 and 6 are disclosed in copending applications Serial Numbers 08/800,698 filed February 14, 1997, 09/146,400 filed September 3, 1998, and 09/146,617 filed September 3, 1998.

Because IgE is believed to mediate allergic symptoms, it may be desirable to decrease IgE levels as a mechanism for alleviating allergic symptoms. However, a patient's own IgE molecules are self-proteins, and immune responses to such proteins are usually suppressed. The suppression of immune responses to self-proteins, i.e., tolerance to self-antigens, is hypothesized to occur in a number of ways.

The current hypothesis for suppression of T cells directed to self-antigens, involves an induction of "clonal deletion" of such T cells in the thymus, whereby T cell receptors which might recognize self-peptides in association with MHC molecules are eliminated, and only those which recognize foreign peptide and MHC molecules are allowed to expand. In addition, suppressor T cells may also exist which prevent the induction of immune responses to self-proteins.

In contrast to the situation with T cells, it is believed that there are many B cells which express receptors (i.e., surface immunoglobulin) for self-proteins, and that the reason these cells do not produce antibodies to self-proteins is because the T cells required for the antigen presentation to the B cell are normally missing.

A B cell which recognizes epitopes (antigen-binding sites) on a patient's own IgE antibodies is capable of generating antibodies, generally IgG, directed to this self-antigen, i.e., IgE. The existence of such B cells, therefore, presents a unique opportunity to induce the production of auto-antibody responses. There is an unmet need for such antibodies in order to treat allergic disease.

The hypothesis regarding "antigen presentation" involves: the recognition of antigen by surface immunoglobulin on the B cell, the internalization and processing of this antigen, the association of peptides derived from the antigen with MHC molecules expressed on the surface of the B cell, and then, the recognition of the associated antigen peptide and MHC molecules by a particular T cell. The T-cell:B-cell interaction then leads to signal transduction in both cells and the synthesis and elaboration of soluble cytokines which eventually result in antibody production by the B cell.

Thus, in most circumstances, only when an antigen is foreign does an immune response occur; otherwise the internalization and processing of self-proteins would regularly lead to the presentation of self peptide-MHC complexes to T cells and thereby lead to autoimmune antibodies.

Therefore, in order to induce an antibody response to a self-peptide, such as IgE, the immune system must be manipulated so as to allow an auto-reactive B cell to become an antibody-secreting B cell. There is an unmet need to manipulate the immune system in this way, particularly in the context of allergic disease.

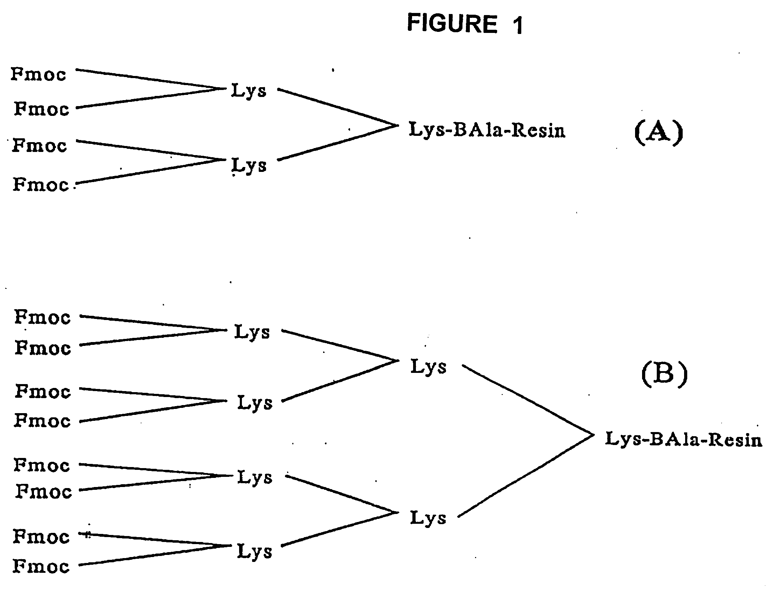

In general, there are several known approaches for generating antibodies to peptide antigens. For example, multiple antigenic peptides (MAPs), introduced by Dr. James Tam (Tam, J.P., (1988) Proc. Natl. Acad. Sci. U.S.A. *85*, 5409-5413), have demonstrated several advantages for inducing anti-peptide antibodies. The MAP approach is an improved alternative to the conventional technique of conjugating a peptide antigen to a protein carrier. One of the primary limitations associated with the use of protein carriers is the large mass of the carrier relative to the attached peptide antigen. This relative size disparity may result in a low ratio of anti-peptide antibodies compared to anti-carrier antibodies. MAPs typically have 4 or 8 peptide arms branching out from a lysine core matrix as depicted in Fig.1 A-B. The peptide antigen is conjugated to each arm. Thus there is a much higher ratio of antigen to carrier molecule in a MAP system compared to traditional protein conjugation. This design maximizes the concentration of the antigen for a specific immunogenic response. Moreover, the central lysine core of the MAP-peptide has been shown to be non-immunogenic. (Tam, J.P., Proc. Natl. Acad. Sci. U.S.A. *85*, 5409-5413 (1988); Posnett, D.N., McGrath, H., and Tam, J.P., J. Biol. Chem*. 263*, 1719-1725 (1988)) Therefore, antibodies induced to MAP-peptides are a direct response to the antigen. Accurate knowledge of the chemical composition, structure, and quantity of the peptide prior to immunization is possible by directly synthesizing the antigen onto the branching lysine core. Also, because the MAP approach removes the need to conjugate peptides to carrier proteins, which may alter the antigenic determinants, chemical ambiguity is eliminated. Thus MAPs are believed to induce antibody responses of high purity, increased avidity, accurate chemical definition, and improved safety.

Fmoc MAP resins (available from Applied Biosystems, Foster City, Calif.) are Fmoc-compatible resins connected to a small core matrix of branching lysine residues. The core matrix comprises several levels of lysine residues attached to the previous lysine at both the N-α and N-ε amino groups, as depicted in Figure 1A-B.

MAP-peptides used in experimental vaccine design have elicited high titers of anti-peptide antibodies that recognize the native protein. (Tam, J.P., (1988) Proc. Natl. Acad. Sci. U.S.A. *85*, 5409-5413; Posnett, D.N., McGrath, H., and Tam, J.P., (1988) J. Biol. Chem. *263*, 1719-1725; Auriault, C., Wolowczuk, I., Gras-Masse, H., Maguerite, M., Boulanger, D., Capron, A., and Tartar, A., (1991) Peptide Res. *4*, 6-11). Additionally, increased sensitivity and reliability of antibody-antigen interactions in solid-phase immunoassays have been observed with MAP-peptides due to enhanced coating capacity and avidity. (Tam, J.P., and Zavala, F., (1989) J. Immunol. Meth., 124, 53-61).

An additional approach that is known to be useful for generating antibodies to peptide antigens involves placing multiple copies of peptides on the surface of plant virus particles. EPICOAT™ technology (Axis Genetics plc, Cambridge, England) is one such example. The EPICOAT™ technology is based on chimeric virus particle (CVP) technology that utilizes the recombinant genetic modification of plant viruses.

The EPICOAT™ technology involves insertion of a small portion of a foreign protein (a peptide) into a plant virus in such a way that multiple copies of the peptide are displayed on the surface of the virus particle. The EPICOAT™ technology is currently based on the cow pea mosaic virus (CPMV) a plant virus that infects the cow pea plant, also known as the "black-eyed" bean. The unmodified CPMV particle is icosahedral, and about twenty-eight nanometers (nm.) in diameter. CPMV particles are composed of two proteins, referred to as the large and small coat proteins. Studies have revealed a site within the small coat protein which allows presentation of a foreign peptide in a prominent position on the virus surface, whereby up to sixty copies of a particular peptide can be presented on each virus particle.

With the EPICOAT™ technology, DNA copies of the plant virus's genetic material are used. A minute quantity of the DNA encoding the virus protein, including the inserted foreign peptide, is applied to the leaves of young cow pea plant, along with an abrasive powder. Upon gentle rubbing, DNA enters the leaves and utilizes the plant's own cellular mechanisms to initiate generation of functional virus particles. The virus replicates within the inoculated leaves and spreads throughout the growing plant.

After two to three weeks, leaf material containing large quantities of the virus is harvested. Chimeric virus particles (CVPs) are isolated by centrifugation and selective precipitation of homogenized plant material. Between 1 to 2 grams of CVPs can be obtained per kilogram fresh weight of leaf material. Cow pea plants are readily grown in abundance in controlled environments, allowing generation of large quantities of CVPs.

It has been reported that peptides of up to thirty-six amino acids in size have been successfully incorporated into CVPs. The resulting particles are extremely robust with a thermal inactivation point of 65°C. The CVPs have been shown to withstand acidic pH as well as protein degrading enzymes. It has been reported that the expressed peptides are capable of eliciting specific immune responses in animals. It has been hypothesized that surface presentation of peptides may enhance the recognition by the host immune system, and may provide a route for development of recombinant sub-unit vaccines and immuno-therapeutics.

### DISCLOSURE OF THE INVENTION

Disclosed is an isolated and purified peptide comprising a cysteine positioned two peptide bonds away from a proline-histidine pair positioned three peptide bonds from a cysteine; the peptide can have the form: SEQ ID NO:1 cysteine-blank-blank-proline-histidine-blank-blank-blank-cysteine. The cysteines may form a covalent bond through a reduction reaction, cyclizing the peptide and becoming cystine. A peptide in accordance with the invention can comprise from 9 to 71 amino, acids.

Disclosed is an isolated and purified peptide comprising a cysteine positioned one peptide bond from a proline-histidine pair, positioned one peptide bond from a proline, positioned two peptide bonds from a cysteine; the peptide can have the form: SEQ ID NO:2 cysteine-blank-proline-histidine-blank-proline-blank-blank-cysteine. The cysteines may form a covalent bond through a reduction reaction, cyclizing the peptide and becoming cystine.

Disclosed is a peptide in accordance with the invention that has the amino acid sequence, or a conservative variant thereof, which comprises: SEQ ID NO:3 serine-valine-threonine-leucine-cysteine-proline-asparagine-proline-histidine-isoleucine-proline-methionine-cysteine-glycine-glycine-glycine. The cysteines may form a covalent bond through a reduction reaction, cyclizing the peptide and becoming cystine.

Disclosed is a peptide in accordance with the invention which has the amino acid sequence, or a conservative variant thereof, which comprises: SEQ ID NO:4 serine-alanine-cysteine-histidine-proline-histidine-leucine-proline-lysine-serine-cysteine-glycine-glycine-glycine. The cysteines may form a covalent bond through a reduction reaction, cyclizing the peptide and becoming cystine.

Disclosed is a peptide in accordance with the invention which has the amino acid sequence, or a conservative variant thereof, which comprises: SEQ ID NO:5 serine-alanine-cysteine-proline-asparagine-proline-histidine-asparagine-proline-tyrosine--cysteine-glycine-glycine-glycine. The cysteines may form a covalent bond through a reduction reaction, cyclizing the peptide changing the amino acid to cystine.

Disclosed is an isolated and purified peptide comprising a leucine positioned two peptide bonds away from a tyrosinearginine pair, or a conservative variant thereof; the peptide can have the form: SEQ ID NO:6 leucine-blank-blank-tyrosine-arginine, SEQ ID NO:7 tyrosine-arginine-blank-blank-leucine, or, SEQ ID NO:8 leucine-blank-blank-tyrosine-arginine-blank-blank-leucine, where blank is any amino acid. At least one blank is preferably an amino acid with an aromatic ring. A peptide in accordance with the invention can comprise from 5 to 71 amino acids.

Disclosed is a peptide in accordance with the invention that has the amino acid sequence, or a conservative variant thereof, which comprises: SEQ ID NO:9 threonine-leucine-leucine-glutamic acid-tyrosine-arginine-methionine.

Disclosed is a peptide in accordance with the invention that has the amino acid sequence, or a conservative variant thereof, which comprises: SEQ ID NO:10 glycine-methionine-asparagine- leucine-threonine-tryptophan-tyrosine-arginine-glutamic acid-serine-lysine.

A peptide in accordance with the invention, or a conservative variant thereof, can be linked to a multiply antigenic peptide which comprises multiple copies of the peptide, or various peptides in accordance with the invention; the multiply antigenic peptide can comprise multiple copies of conservative variants of peptides in accordance with the invention.

A peptide in accordance with the invention, or a conservative variant thereof, can be linked to a plant virus particle which comprises multiple copies of the peptide, or various peptides in accordance with the invention; the plant virus particle can comprise multiple copies of conservative variants of peptides in accordance with the invention.

Disclosed is a method for generating canine auto-antibodies directed to the canine IgE molecule, said method comprising providing a peptide in accordance with the invention or a conservative variant thereof, and administering the provided peptide to a dog. The method can further comprise a step of mixing the provided peptide with an adjuvant prior to the administering step, wherein the administering step comprises administering the mixture of the peptide and the adjuvant. The mixing step can comprise mixing the peptide with the adjuvant at a weight ratio of about one part peptide to five parts adjuvant. The method can further comprise a step of administering a booster dose of the provided peptide, following the administering step. Treatment or prophylaxis of canine allergy is accomplished by performing a method in accordance with the invention.

Also disclosed is an immunogenic molecule comprising a peptide in accordance with the invention or a conservative variant of a peptide in accordance with the invention linked to a carrier molecule capable of facilitating an immune response. For example, the carrier molecule can be KLH, BSA, MLA, a multiply antigenic peptide, or a plant virus particle.

Also disclosed are peptides that specifically bind a peptide in accordance with the invention; disclosed are peptides that are raised to and specifically bind a peptide in accordance with the invention.

### DEFINITIONS

### Amino Acids:

| **AMINO ACID ABBREVIATIONS** | | |
|---|---|---|
| **Amino Acid** | **One-Letter Symbol** | **Three-Letter Symol** |
| alanine | A | ala |
| arginine | R | arg |
| asparagine | N | asn |
| aspartic acid | D | asp |
| cysteine | C | cys |
| glutamic acid | E | glu |
| glutamine | Q | gln |
| glycine | G | gly |
| histidine | H | his |
| isoleucine | I | ile |
| leucine | L | leu |
| lysine | K | lys |
| methionine | M | met |
| phenylalanine | F | phe |
| proline | P | pro |
| serine | S | ser |
| threonine | T | thr |
| tryptophan | W | trp |
| tyrosine | Y | tyr |
| valine | V | val |

*cDNA* clone: A duplex DNA sequence representing an RNA, carried in a cloning vector.
*Cloning*: The selection and propagation of a single DNA species.
*Cloning Vector*: A plasmid, phage DNA or other DNA sequence, able to replicate in a host cell and capable of carrying exogenously added DNA sequence for purposes of amplification or expression of the added DNA sequence.
*Codon:* A triplet of nucleotides that represents an amino acid or termination signal.
*Conservative variants:* Conservative variants of nucleotide sequences include nucleotide substitutions that do not result in changes in the amino acid sequence encoded by such nucleotides, as well as nucleotide substitutions that result in conservative amino acid substitutions, e.g., amino acid substitutions which do not substantially affect the character of the polypeptide translated from said nucleotides. For example, the character of a peptide derived from IgE is not substantially affected if the substitutions do not preclude specific binding of the peptide to canine IgE receptor or other canine IgE binding ligands.
   Conservative variants of amino acid sequences include amino acid substitutions or deletions that do not substantially affect the character of the variant polypeptide relative to the starting peptide. For example, polypeptide character is not substantially affected if the substitutions or deletions do not preclude specific binding of the variant peptide to a specific binding partner of the starting peptide. The term mimotope refers to a conservative variant of an amino acid sequence, to which antibody specificity has been raised. The mimotope comprises a variant of the epitope of the starting peptide such that it is able to bind antibodies that cross-react with the original epitope.
*DNA Sequence:* A linear series of nucleotides connected one to the other by phosphodiester bonds between the 3' and 5' carbons of adjacent pentoses.
*Expression:* The process undergone by a structural gene to produce a polypeptide. It is a combination of transcription and translation.
*Expression Control Sequence:* A DNA sequence of nucleotides that controls and regulates expression of structural genes when operatively linked to those genes.
*Exon:* A contiguous region of DNA encoding a portion of a polypeptide. Reference to any exon, e.g. "DNA sequence of exon 6", refers to the complete exon or any portion thereof.
*Genome:* The entire DNA of a substance. It includes *inter alia* the structural genes encoding for the polypeptides of the substance, as well as operator, promoter and ribosome binding and interaction sequences such as the Shine-Dalgarno sequences.
*Nucleotide:* A monomeric unit of DNA or RNA consisting of a sugar moiety (pentose), a phosphate, and a nitrogenous heterocyclic base. The four DNA bases are adenine ("A"), guanine ("G"), cytosine ("C") and thymine ("T") . The four RNA bases are A, G, C and uracil ("U"). A and G are purines, and C, T, and U are pyrimidines.
*Phage or Bacteriophage:* Bacterial virus, many of which include DNA sequences encapsidated in a protein envelope or coat ("capsid").
*Plasmid:* An autonomous self-replicating extrachromosomal circular DNA.
*Polymerase Chain Reaction (PCR):* A method of amplifying a target DNA sequence contained in a mixture of DNA sequences, by using oligonucleotide primers that flank the target DNA sequence for repeated cycles of DNA synthesis of the target DNA sequence.
*Polypeptide:* A linear series of amino acids connected one to the other by peptide bonds between the a-amino and carboxyl groups of adjacent amino acids.
*Reading Frame:* The grouping of codons during translation of mRNA into amino acid sequences. For example, the sequence GCTGGTGTAAG may be translated in three reading frames or phases, each of which affords a different amino acid sequence:
   GCT GGT TGT AAG-Ala-Gly-Cys-Lys
   G CTG GTT GTA AG-Leu-Val-Val
   GC TGG TTG TAA A-Trp-Leu-(STOP).
*Recombinant DNA Molecule:* A hybrid DNA sequence comprising at least two nucleotide sequences, the first sequence not normally being found together in nature with the second.
*Specific binding*: Binding of one substance to another at greater binding affinity than background binding. Two substances that exhibit specific binding are referred to as specific binding partners, or as a specific binding pair. An antibody and its antigen are one example of a specific binding pair.
*Specific Binding Molecule:* A molecule that exhibits specific binding to its corresponding binding partner to form a specific binding pair. As used herein, this definition of specific binding molecule covers monoclonal and polyclonal antibodies, antigen-binding fragments of these antibodies, hybrid antibodies, single-chain antibodies, and recombinant molecules capable of specific binding to a ligand.
*Structural Gene:* A DNA sequence that encodes through its template or messenger RNA ("mRNAII) a sequence of amino acids characteristic of a specific polypeptide.
*Transcription:* Synthesis of RNA on a DNA template.
*Translation:* Synthesis of peptides on the mRNA template.

### DESCRIPTION OF FIGURES

Fig. 1A depicts the core structure of a MAP protein with four arms, i.e., a 4-MAP protein; Fig. 1B depicts the core structure of a MAP protein with eight arms, i.e., an 8-MAP protein.
Fig. 2 depicts data comparing the binding characteristics of several monoclonal antibodies to surface bound IgE (e.g., analogous to IgE expressed on the surface of B cells); IgE receptor (e.g., analogous to the Fc receptor on mast cells); and to a combination of IgE when bound by the IgE receptor. Thus each component assayed was immobilized on a solid surface.
Fig. 3 depicts the ability of recombinant exon 3 to inhibit conjugated antibody 8H.8 or conjugated antibody 15A.2 from binding to an IgE solid phase. Data for 8H.8 is depicted by speckled bar graphs, data for 15A.2 is depicted by solid black bar graphs.
Fig. 4 depicts the ability of soluble, purified canine IgE to inhibit monoclonal antibody 8H.8 (2.5 µg/ml) or monoclonal antibody 15A.2 (2.5 µg/m1) from binding to a canine IgE solid phase. Data for 8H.8 is depicted by speckled bar graphs; data for 15A.2 is depicted by solid black bar graphs.
Fig. 5 depicts the ability of monoclonal antibody 15A.2 or monoclonal antibody 8H.8 to inhibit IgE from binding to recombinant IgE receptor immobilized on a solid phase as detected by monoclonal antibody D9.
Fig. 6 depicts the amino acid sequences of the New England Biolabs PhDc7c phage display library that were bound by the monoclonal antibody 15A.2. These sequences are shown in alignment with the protein sequence of canine IgE.
Fig. 7 depicts the alignment of the 15A.2 binding region from seven different mammals: dog, human, green monkey, cat, swine, mouse and horse.
Fig. 8 depicts the ability of phage displaying 15A.2 mimotope peptides to inhibit canine IgE from binding the 15A.2 monoclonal antibody on the solid phase.
Fig. 9 depicts the ability of a specific 15A.2 mimotope peptide sequence attached to the solid phase to be able to bind the 15A.2 mimotope monoclonal antibody.
Fig. 10A and 10B depict the ability of a 15A.2 mimotope peptide to be able to prevent the 15A.2 monoclonal antibody from binding canine IgE on the solid phase.
Fig. 11 depicts data showing serum IgE concentration of immunized dogs. Pre-bleed data is depicted by dark speckled bar graphs, "immunized" data is depicted by solid black bar graphs, "boosted" data is depicted in open bar graphs, and data for samples taken 10.24.97 is depicted in lightly speckled bar graphs.
Fig. 12 depicts the level of auto anti-IgE in dogs over time, for dogs 1 through 4. Data for pre-bleed samples is depicted by darkly speckled bar graphs, data for "immunized" samples is depicted by solid black bar graphs, data for "boosted" samples is depicted by open bar graphs, and data for samples taken 10.24.97 is depicted by lightly speckled bar graphs.
Fig. 13 depicts the ability of immune serum, from dog 4, to inhibit conjugated 8H.8 from binding to native canine IgE immobilized on a solid phase. Data for samples taken "prebleed" is depicted by darkly speckled bar graphs, data for the first bleed samples is depicted by solid black bar graphs, data for control with antibody 14A.3 (10 µg/ml) is depicted by lightly speckled bar graphs and data for control 8H.8 (10 ng/ml) is depicted by medium speckled bar graph.
Fig. 14 depicts the ability of monoclonal antibody 8H.8 and monoclonal antibody 15A.2 to bind to purified canine IgE immobilized on a solid phase.
Fig. 15 depicts data from a study evaluating the ability of antibody 15A.2 conjugated to a signal moiety to bind to solid phases having various peptides immobilized thereon.
Fig. 16 depicts data from a study evaluating the ability of monoclonal antibody 14K2, known to bind with canine IgE exon 4, to bind with various peptides immobilized on a solid phase.
Fig. 17 depicts data from a study evaluating the ability of monoclonal antibody 15A.2, known to bind to canine IgE exon 3, to bind to various peptides immobilized on a solid phase.
Fig. 18 depicts data for studies that compared the binding of monoclonal antibody 8H.8, or a polyclonal antibody raised to recombinant IgE (Re-hu IgE), to bind to a solid phase having peptide E3a.5 or substituted peptide E3a.5 (designated peptide S3a.5) immobilized thereon.
Fig. 19 depicts data from a study evaluating the ability of antibody 8H.8 conjugated to a signal moiety to bind to solid phases having various peptides immobilized thereon.
Fig. 20 depicts the ability of polyclonal anti-human IgE antibodies to bind to various peptides immobilized on a solid phase.

### MODES FOR CARRYING OUT INVENTION

### Proposed Mechanism of Action of Anti-IgE Monoclonal Antibodies in Causing Persistent Serum IgE Depletion

A hypothesis in accordance with the present invention is that anti-IgE monoclonal antibodies affect IgE levels by directly binding with the resident circulating IgE, after which the bound complex is removed from the circulation.

Moreover, it is hypothesized that anti-IgE monoclonal antibodies affect IgE levels by interfering with the production of new IgE by B-cells. Memory B-cells, cells that upon interacting with antigen and T-cell become antibody producing cells (i.e., "memory IgE B-cells"), are involved in replenishing serum IgE, and these cells contain IgE as their cell surface receptors. Thus, it is possible that anti-IgE monoclonal antibodies bind to the IgE on these antibody producing cells and interfere with their function, such as by down-regulation or by one or more mechanisms which lead to cell destruction. Two general ways have been proposed for how the binding of a monoclonal antibody to a memory B cell might interfere with production of IgE.

First, activation of a memory B cell, may also require the involvement of a surface IgE-binding factor designated CD23. Binding of certain monoclonal antibodies to the cell surface IgE may preclude CD23 binding, and may thus lead to an inability to mount an IgE response.

Second, IgE antibody production by a memory IgE B cell may be reduced or eliminated by binding of a monoclonal antibody that is directed against, or blocks, the receptor for the IgE molecule which is located on the surface of the memory B cell.

In the absence of a monoclonal antibody that leads to memory IgE B cell elimination or inactivation, however, it would be expected that the memory IgE B-cells would be replenished, leading to the eventual replenishment of circulating IgE.

The serum levels of IgE are elevated in patients experiencing allergic disease. As disclosed herein, when antibodies were generated that specifically bind to a species' IgE, and these antibodies were administered to a patient who is a member of the species ("passive immunization"), that patient's serum levels of IgE declined. As appreciated by one of ordinary skill in the art, the specific binding proteins in accordance with the invention are administered in pharmacologically accepted dosages, and can be administered with suitable biocompatible diluents. Also disclosed herein are a method and related compositions that serve to induce a response to a self-peptide, such as an IgE molecule, whereby the immune system is manipulated so as to allow an auto-reactive B cell to become an antibody--secreting B cell ("active immunization").

Receptors are present on mast cells that bind to IgE from the circulation. When IgE has become bound to the receptors on mast cells, the IgE molecules can become crosslinked. Upon cross-linking these IgE molecules, the mast cell is induced to release histamine. Histamine is an agent that induces the manifestation of allergic symptoms. Generally, cross-linking occurs by binding of the IgE molecules to an antigen, e.g., an allergen. However, a monoclonal antibody that binds to IgE could serve as a means for cross-linking IgE which has become bound to the surface of mast cells.

For allergic individuals, if anti-IgE antibodies were present in circulation, it becomes easy to envisage that the binding of such auto-anti-IgE to IgE molecules on mast cells could serve to cross-link the IgE molecules on those cells and exacerbate an ongoing allergic response even in the absence of allergen. This occurrence is clearly undesirable in the context of allergy treatment. An antibody that achieved this function would therefore not be preferred for use as a therapeutic in accordance with the invention. Therefore, an antibody that achieves such crosslinking is disadvantageous, and is not preferred in accordance with the invention.

Two approaches exist for producing monoclonal antibodies which target IgE, but which do not cross-link IgE molecules which have become bound to a mast cell. The first is to produce monoclonal antibodies that are directed to an epitope of an IgE molecule that is only accessible when the IgE is in circulation. Since the monoclonal antibody can only bind to IgE when it is in circulation, it will not be able to bind to IgE that has become bound to the surface of a mast cell.

An alternative approach for producing anti-IgE antibodies has been hypothesized by Stanworth et al, e.g., U.S. Patent No. 5, 601, 821 issued 11 February 1997. The antibodies of Stanworth are disclosed to cross-link IgE on mast cells, but in a manner that does not induce histamine release. Stanworth has disclosed an epitope of the human IgE molecule which must be available or accessible after IgE molecules have become cross-linked on the surface of a mast cell, in order for the mast cell to release histamine. Thus, Stanworth disclosed an antibody that binds to that particular epitope. Accordingly, monoclonal antibodies that are directed to this IgE epitope will serve to cross-link the IgE, but will not permit the mast cell to release histamine.

The approach followed in accordance with the present invention is the development of antibodies, either *ex vivo* monoclonal or *in vivo* anti-self antibodies, directed to epitopes which are accessible on circulating IgE but which are not accessible when such IgE becomes bound to a mast cell.

### Passive Immunity

### Monoclonal Antibodies 15A.2 and 8H.8

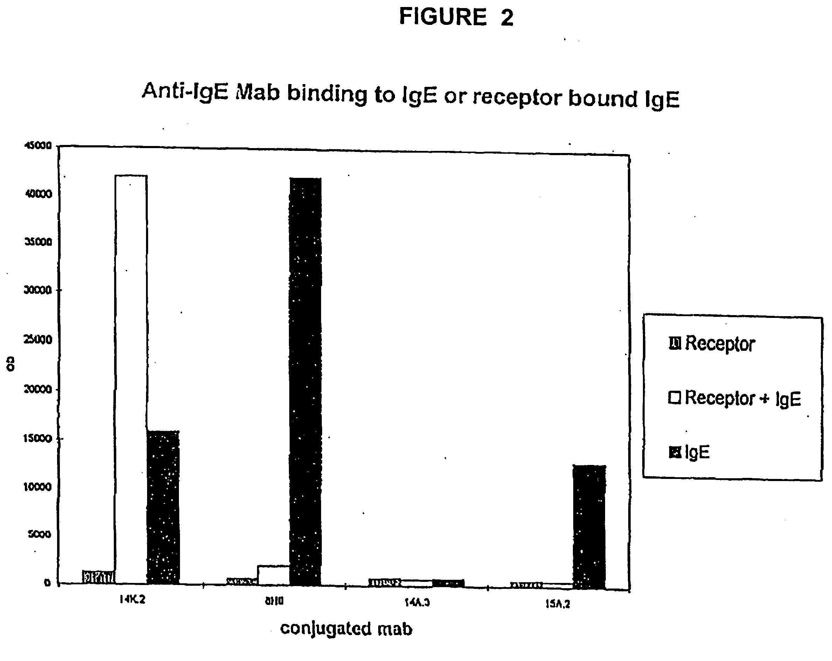

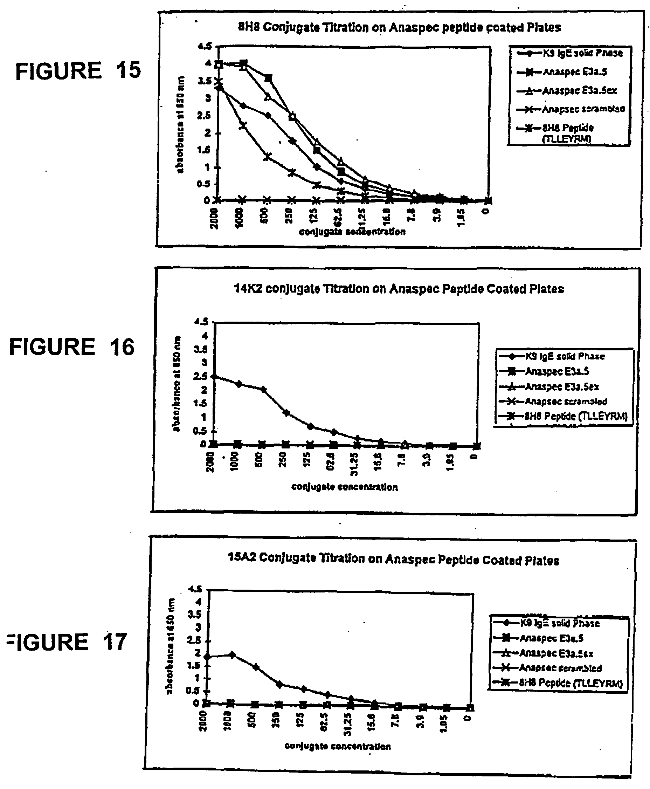

Monoclonal antibody 15A.2 binds to canine IgE. The 15A.2 monoclonal antibody was derived by immunizing mice with affinity-purified canine IgE. The epitope bound by 15A.2 is conformational, not linear. As indicated by data depicted in Figure 2, 15A.2 did not bind to the IgE receptor; nor did it bind to the IgE when bound to receptor. However, 15A.2 exhibited affinity for free IgE. It binds to recombinant canine exon 3 fusion proteins in an enzyme linked immunosorbent assay (ELISA) and by Western blot, but not to other recombinant canine IgE fusion proteins.

Characterization experiments of the 15A.2 monoclonal antibody showed that 15A.2 did not bind to IgE that was already bound by the IgE receptor on mast cells. Thus, it appeared that access to the epitope bound by 15A.2 was hindered by IgE binding to the Fc receptor on mast cells. Accordingly, 15A.2 will not crosslink IgE bound to mast cells. This finding was demonstrated by binding studies with a recombinant receptor discussed herein.

Monoclonal antibody 8H.8 also binds to canine IgE. The 8H.8 monoclonal antibody was derived by immunizing mice with a shortened version of exon 3 of the canine IgE molecule, designated exon 3a. Exon 3a contains the C-terminal 71 amino acids of the full length exon 3. Previous studies (data not presented herein) had shown that immunizing mice with the full length exon 3 did not generate antibodies having specificity such as that ultimately found with the 8H.8 antibody.

Characterization experiments of the 8H.8 monoclonal antibody showed that, unlike monoclonal antibodies derived by immunization with all other recombinant IgE sequences, 8H.8 would also bind to native canine IgE. Additionally, like 15A.2 and as depicted in Figure 2, it was found that 8H.8 did not bind to IgE that was already bound by the IgE receptor on mast cells. Thus, it appeared that access to the epitope bound by 8H.8 was also hindered by IgE binding to the Fc receptor on mast cells. As a result, 8H.8 will not crosslink IgE bound to mast cells. This finding was demonstrated by binding studies with a recombinant receptor discussed herein.

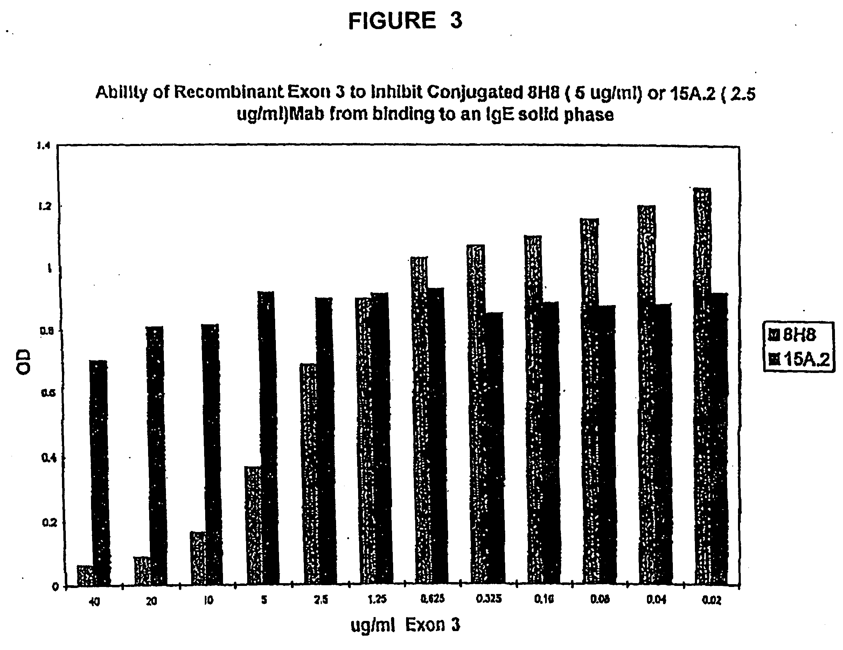

Competitive assays were performed between an antibody, 8H.8 or 15A.2, and soluble exon 3 to identify the level of inhibition of binding of these antibodies to affinity purified native IgE immobilized on the solid phase of an ELISA. The results from this study are depicted in Figure 3. In Figure 3 it is seen that increasing the concentration of recombinant exon 3 inhibited the binding of monoclonal antibody 8H.8, but did not inhibit antibody 15A.2.

In addition, the ability of these monoclonal antibodies to inhibit IgE from binding to recombinant IgE receptor on an ELISA solid phase was examined.

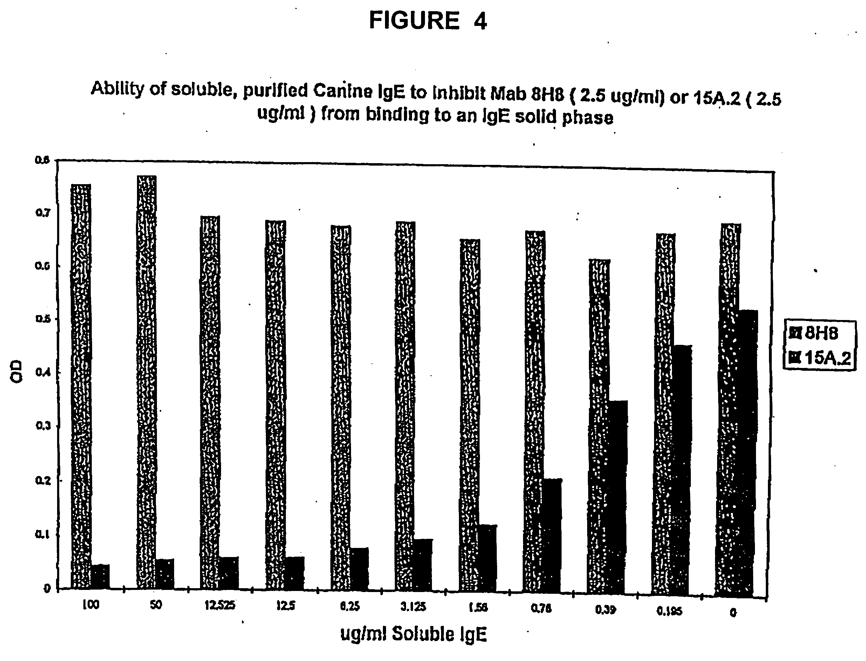

Additionally, competitive assays were performed with antibody 8H.8, and analogous assays were performed with 15A.2, where the antibody was in competition with affinity-purified canine IgE in solution and IgE on an ELISA solid phase. Data from these assays is depicted in Figure 4.

These studies suggested that 15A.2 had a high affinity for soluble native canine IgE because as the concentration of the soluble IgE was increased, the binding of 15A.2 to the immobilized immunoglobulin dropped appreciably, indicating that 15A.2 was now binding to the soluble IgE. In contrast, it is seen that antibody 8H.8 has a much lower affinity for soluble IgE, because increasingly high concentrations of the soluble IgE did not inhibit the ability of 8H.8 to bind to the immobilized IgE. Thus, the data suggest that soluble IgE was not effective at inhibiting 8H.8 from binding to IgE immobilized on a solid phase. Thus, the affinity of 8H.8 for soluble canine IgE was lower than the affinity of this monoclonal antibody for IgE on a solid phase.

These studies further suggested that 8H.8 had a low affinity for soluble native canine IgE, because increasingly high concentrations of the soluble IgE did not inhibit the ability of 8H.8 to bind to the immobilized IgE. In contrast, it is seen that antibody 15A.2 has a much higher affinity for soluble IgE, since as the concentration of the soluble IgE was increased, the binding of 15A.2 to the immobilized immunoglobulin dropped appreciably, indicating that 15A.2 was now binding to the soluble IgE. Thus, the data suggested that soluble IgE was not effective at inhibiting 8H.8 from binding to IgE immobilized on a solid phase. Thus, the affinity of 8H.8 for soluble canine IgE was lower than the affinity of this monoclonal for IgE on a solid phase.

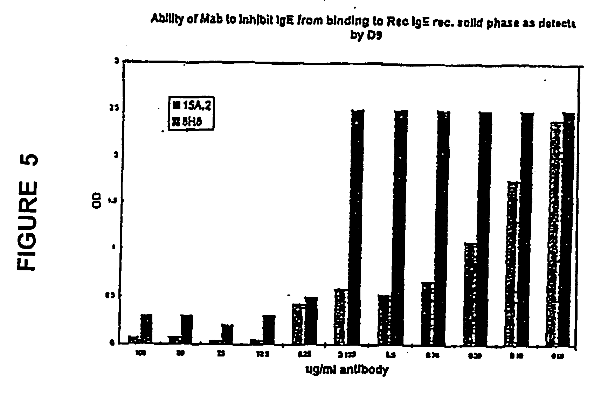

To further substantiate the finding that 15A.2 has a higher affinity for soluble native IgE, and that 8H.8 had a low affinity for soluble native IgE, studies were performed where 8H.8 was used, and analogous studies were conducted with 15A.2, to determine the extent to which each antibody inhibits binding of the soluble IgE to a recombinant IgE receptor solid phase. A labeled antibody D9 was used as a means for detection in the studies depicted in Figure 5. The monoclonal antibody D9 is known to bind to IgE when bound to receptor. For the studies depicted in Figure 5, the native IgE was present at a concentration of 2.5 micrograms per milliliter. As shown in Figure 5, 15A.2 was effective at inhibiting binding of the native IgE to recombinant IgE receptor at antibody concentrations as low as 190 nanograms per milliliter. In contrast, 3,125 nanograms per milliliter of 8H.8 were necessary to inhibit the binding of native IgE to the recombinant IgE receptor. Thus, since more 8H.8 than 15A.2 was required to inhibit the binding of native IgE to the recombinant receptor, it was seen that 8H.8 had a lower affinity for the soluble IgE. It appears, therefore, that when a sufficient concentration of either 8H.8 or 15A.2 antibodies is provided, that the antibodies impair the binding of soluble IgE to the IgE receptor.

Furthermore, as indicated by the data depicted in Figure 2, the avidity of 8H.8 binding for native canine IgE was greatly increased when the IgE was immobilized. These studies suggested that 8H.8 had a low affinity for soluble native canine IgE, but that when the IgE is immobilized on a surface, or when it is expressed on the surface of a memory B cell, the avidity of 8H.8 binding for native canine IgE was greatly increased.

Accordingly, in view of these findings, it was hypothesized that the region within native IgE recognized by 8H.8 might be partially hidden, particularly when the IgE is in serum. A partially sequestered amino acid sequence of IgE may not be readily exposed to the native canine immune system and its normal protective mechanisms. Furthermore, since the full length exon 3 did not lead to the generation of antibodies having 8H.8-like specificity, the full length sequence might contain suppressor peptides capable of down-regulating or eliminating an auto-anti-IgE response which is specific against the epitope recognized by the 8H.8 antibody. Either mechanism could serve to avoid production of an autoimmune response to self-antigens.

The implications of these findings are that 8H.8 antibody, when used as an allergy therapeutic in dogs, would preferentially bind to IgE on memory B cells rather than to IgE in solution. Moreover, 8H.8 has a low binding affinity for IgE when bound by Fc receptor on mast cells.

Phage display technology was used to map the epitope bound by the 8H.8 antibody. A preferred method for performing phage display technology is accomplished by use of a Ph.D. Phage Display Peptide Library Kit (New England BioLabs, Beverly Mass.). It was found that a 7 amino acid peptide (Thr-Leu-Leu-Glu-Tyr-Arg-Met) inhibited monoclonal antibody 8H.8 from competitive binding to either native or recombinant canine IgE. This sequence contained 6 amino acids common in form and/or spacing to the C-terminal region of exon 3. An 11 amino acid peptide synthesized from this region (SEQ ID NO:10 Gly-Met-Asn-Leu-Thr-Trp-Tyr-Arg-Glu-Ser-Lys) designated E3a. 5 also inhibited monoclonal antibody 8H.8 from competitive binding to native or recombinant IgE.

It is believed that antibody responses with specificity such as that of 8H.8 do not occur naturally, even upon occurrence of events which would induce auto-anti-IgE responses to other epitopes, because the 8H.8 epitope is only partially available for recognition. Nevertheless, it is hypothesized that antibodies to an 8H.8-type epitope, generated from peptide immunization in accordance with the invention do bind to the partially available epitope, as does the 8H.8 monoclonal antibody.

It is further hypothesized that the region within native IgE recognized by 15A.2 might serve as an immunogen to induce auto-anti-IgE responses and might generate antibodies capable of binding to IgE+ B cells and of down regulating IgE synthesis. The implications of these findings are that 15A.2 antibody, when used as an allergy therapeutic in dogs, would prevent IgE from binding to mast cells and potentially affect the synthesis of IgE by B cells.

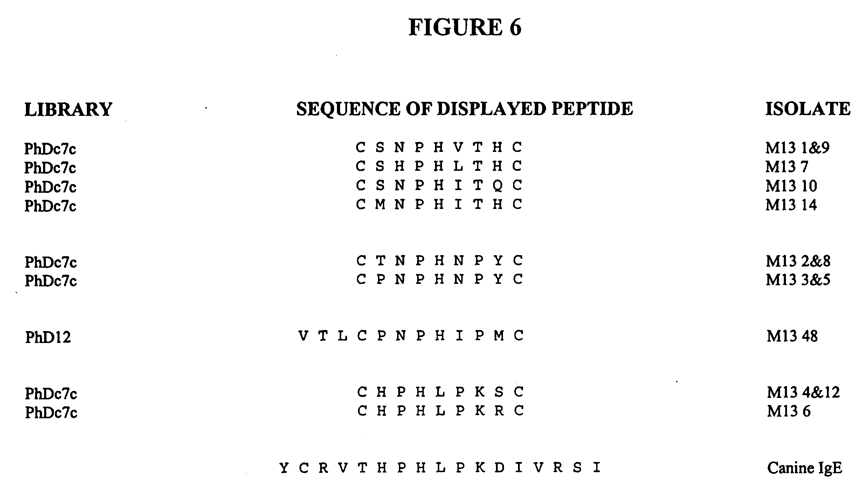

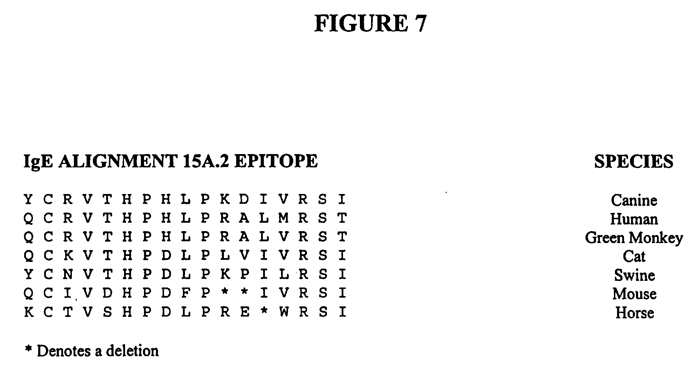

Phage display technology was used to map the epitope bound by the 15A.2 antibody. As noted above, a preferred method for performing phage display technology is accomplished by use of a Ph.D. Phage Display Peptide Library Kit (New England BioLabs, Beverly Mass.). Figure 6 depicts the amino acid sequences of the PhDc7c library that were bound by the monoclonal antibody 15A.2. These sequences are shown in alignment with the protein sequence of canine IgE. Figure 7 depicts the alignment of the 15A.2 epitope region from seven different mammals: dog, human, green monkey, cat, swine, mouse and horse.

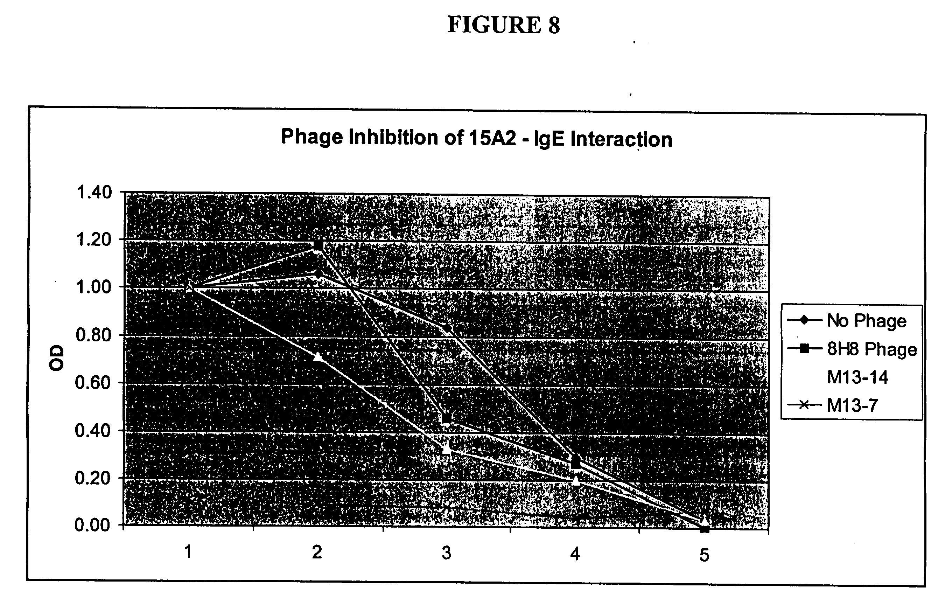

Figure 8 portrays the ability of different phage displaying 15A.2 mimotope peptides to inhibit canine IgE from binding the 15A.2 monoclonal antibody on the solid phase. 466 µl of biotinylated 15A.2 antibody (10 µg/ml) were mixed with 466 µl of phage from a fresh overnight culture. Into each well of a microtiter plate 100 µl of the mixture was added. The antibodies and phage were allowed to bind for 2 ½ hours. The wells were then coated with strepavidin. The plates were washed three times with standard wash buffer to remove loosely bound material. A serial dilution of canine IgE was prepared, starting with the concentration of 1 µg/100 µl of PBS, o.1% Tween. 100 µl of dilution IgE was added per well and allowed to bind for 10 minutes. The competition reaction was stopped by washing the plates five times with wash solution. The plate was then developed with HRPO linked D9 monoclonal antibody, which binds to domain 4 of IgE, to visualize the IgE in the solid phase. Loss of signal indicates the phage successfully repelled canine IgE competition. Table 1 shows the concentrations of the reactants at the various points on the x-axis of figure 8.

**TABLE 1**

| | **IgE** | **phage** |
|---|---|---|
| 1 | 1.0 µg | 0 |
| 2 | 1.0 µg | 50 µl |
| 3 | 0.5 µg | 50 µl |
| 1 | 1.0 µg | 0 |
| 4 | 0.25 µg | 50 µl |
| 5 | 0.125 µg | 50 µl |

Using the New England Biolabs PhD12 phage display library, it was found that a 12 amino acid peptide (SEQ ID NO: 11 Val-Thr-Leu-Cys-Pro-Asn-Pro-His-Ile-Pro-Met-Cys) inhibited monoclonal antibody 15A.2 from competitive binding to either native or recombinant canine IgE. This sequence contained 4 amino acids common in form and/or spacing to the N-terminal region of exon 3.

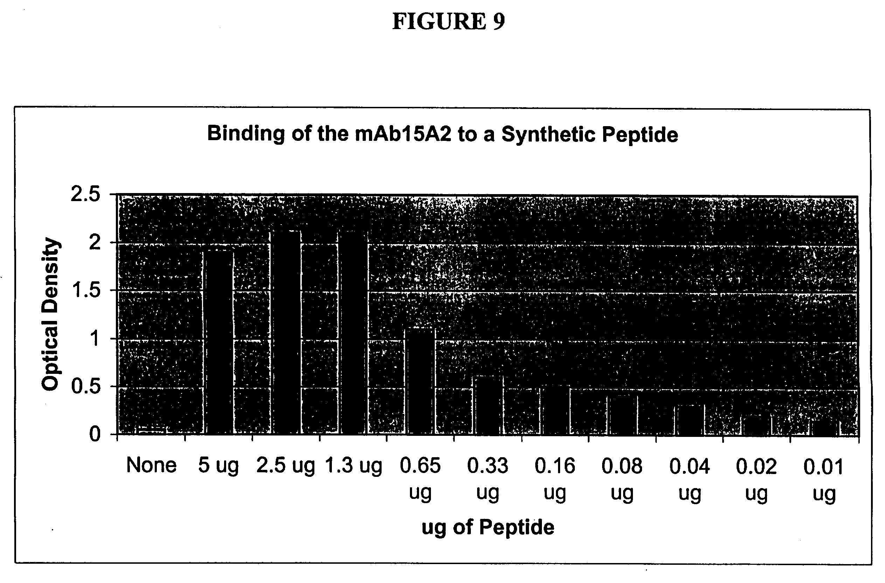

Figure 9 displays the ability of 15A.2 monoclonal antibody to bind a synthetic peptide. The peptide SEQ ID NO: 3 Ser-Val-Thr-Leu-Cys-Pro-Asn-Pro-His-Ile-Pro-Met-Cys-Gly-Gly-Gly-Lys was synthesized and biotinylated on the epsilon carbon of the Lys residue. This sequence corresponds to the isolate M13-48. The peptide was treated in a manner to promote reduction and cyclization of the cysteines to form a cyclic peptide. A serial dilution of the peptide was prepared and bound to strepavidin-coated microtiter plates (5 µg strepavidin per well). Bound peptide was detected with HRPO conjugated 15A.2 monoclonal antibody. Figure 9 shows the results of this experiment. This solid phase specific 15A.2 mimotope peptide bound the 15A.2 monoclonal antibody in a concentration dependent manner.

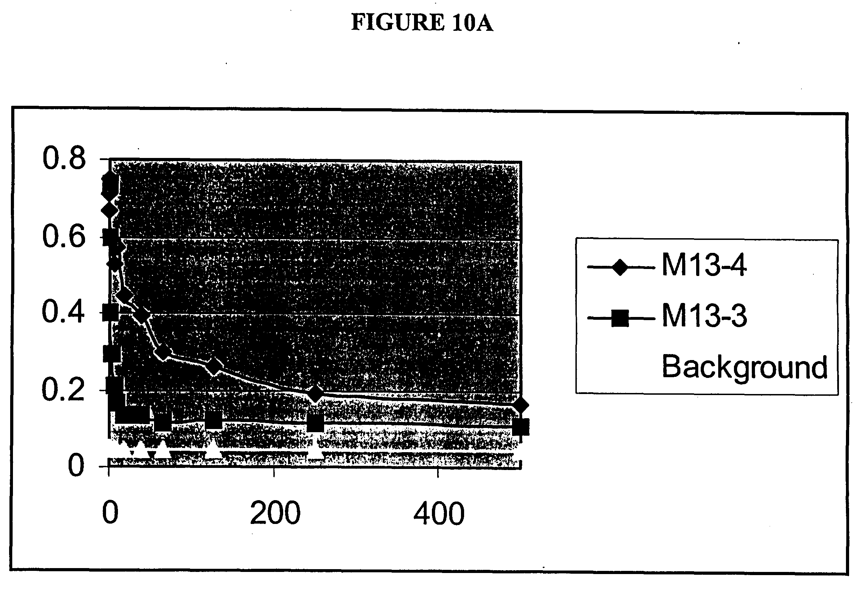

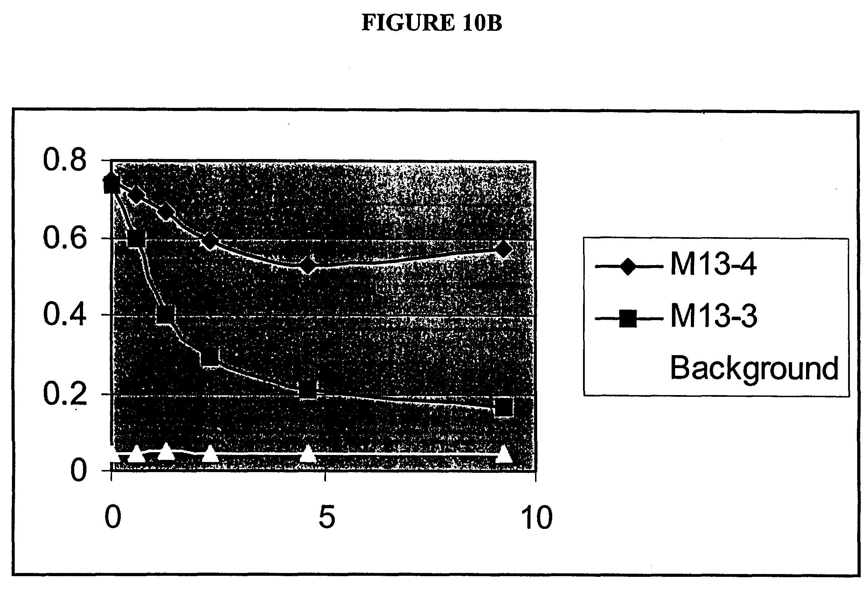

Figures 10A and 10B depict the ability of a 15A.2 mimotope peptide to prevent the 15A.2 monoclonal antibody from binding canine IgE on the solid phase. Plates were coated with canine IgE at a concentration of 1 µg/ml. A serial dilution of the synthetic peptide was added to HRPO conjugated 15A.2 monoclonal antibody (final concentration 1 µg/ml) and allowed to bind for two hours. 100 µl of the 15A.2/peptide mixture was added to the wells and allowed to bind 1 hour. The reaction was stopped by washing the plates six times with wash buffer. The plates were developed with HRPO substrate, the reactions stopped with stop mix, and the plates were measured for O.D. using a plate reader.

Apart from active immunization with antibody 15A.2 or 8H.8 to induce auto-anti-IgE production *in vivo*, this invention also comprises a specific binding molecule that specifically binds a ligand of the type bound by 15A.2 or 8H.8, as well as the therapeutic or prophylactic use thereof. Thus, the invention comprises a monoclonal antibody specific for a conservative variant of a sequence bound by 15A.2, for a conservative variant of a sequence bound by 8H.8, or for a native sequence of canine IgE up to 100 amino acids from the C' terminal portion of exon 3. The specific binding molecules of the invention can be used in a method in accordance with the invention as a treatment for or as prophylaxis for allergy symptoms, i.e., passive immunization.

Accordingly, administration to a dog of either an antibody in accordance with the invention, such as 15A.2 or 8H.8, or a peptide in accordance with the invention, such as the 7, 11 or 12 amino acid peptide, leads to a diminution of further IgE production. This may occur, for example, by the 15A.2 or 8H.8 antibody binding to the memory B cell and preventing further IgE production. For a peptide, its administration would lead to production of antibodies of comparable specificity and effect as 15A.2 or 8H.8.

### Active Immunity

It is possible to generate an auto-anti-IgE response by immunizing an animal with a peptide derived from the sequence of its own IgE, along with a peptide that might associate with an MHC molecule. The size and sequence of the IgE-derived peptide and/or the product of its internal processing in antigen presenting B cells may represent a unique sequence, which when associated with MHC effectively appears foreign to T cells. A unique auto-anti-IgE specificity may, therefore, be generated. When the animal generates this response and produces antibodies that behave as 15A.2 or 8H.8. The resulting immune response serves to target the IgE-expressing B cell. This targeting affects IgE synthesis by down regulating IgE synthesis from B cells, and/or targets the IgE+ B cell for destruction.

Thus, in accordance with the present invention, a preferred sequence of eleven amino acids was identified, this sequence corresponded to a native expressed amino acid sequence from the C-terminal region of exon 3 of canine IgE. In a preferred embodiment, the present invention comprises an 11 amino acid peptide with the sequence SEQ ID NO:10 Gly-Met-Asn-Leu-Thr-Trp-Tyr-Arg-Glu-Ser-Lys.

Alternative preferred embodiments in accordance with the invention comprise an isolated and purified peptide of five or more amino acids which when administered to a dog, either alone or with an adjuvant or carrier, induce the production of antibodies directed to that dog's IgE when in solution but with low binding affinity when the IgE is bound to mast cells. Thus, in one preferred embodiment, a native sequence of amino acids from the C-terminal region of canine IgE exon 3, of from 5 to 71 amino acids, or a conservative variant of such a sequence, is used. Particularly preferred is an 11 amino acid stretch of exon 3, designated peptide E3a.5.

All peptides, including native sequences and conservative variant sequences derived from peptides which bind the 8H.8 monoclonal antibody preferably contain at least one leucine positioned two peptide bonds away from a tyrosine-arginine pair, i.e., contain a sequence in the form of: SEQ ID NO:6 L-blank-blank-Y-R, SEQ ID NO:7 Y-R-blank-blank-L, or, SEQ ID NO:8 L-blank-blank-YR-blank-blank-L. Blank can be any amino acid; preferably at least one blank in a particular sequence is an amino acid with an aromatic ring.

Accordingly, a host is actively immunized against allergic manifestations by administration of a peptide containing molecule which is able to induce the formation of antibody capable of binding circulating IgE and/or down regulating the synthesis of endogenous IgE. This reduction in the serum level of IgE reduces, or eliminates, allergic responses in these animals. For the first time in the art, it was found that an amino acid sequence in accordance with the invention induced an auto-anti-IgE response in dogs when they were immunized with the sequence. The auto-anti-IgE response reduced the level of serum IgE measured in these dogs. This reduction in the serum level of IgE reduces, or eliminates, allergic responses in these animals.

Therefore, in accordance with the present invention, a defined auto-anti-IgE response was achieved upon administration of the peptides in accordance with the invention. This auto-anti-IgE response decreased the serum IgE levels in the recipient dogs. The identified peptide is used to induce these responses in puppies as a prophylaxis for allergy. In an alternative embodiment, these peptides are administered to any dog that is experiencing clinical manifestations of allergy. For example, peptides in accordance with the invention are administered to those dogs experiencing allergic dermatitis.

Alternative preferred embodiments in accordance with the invention comprise an isolated and purified peptide of 12 or more amino acids which when administered to a dog, either alone or with an adjuvant or carrier, induces the production of antibodies directed to that dog's IgE when in solution. Thus, in one preferred embodiment, a sequence of amino acids designated SEQ ID NO:11 Val-Thr-Leu-Cys-Pro-Asn-Pro-His-Ile-Pro-Met-Cys, or a conservative variant of such a sequence, is used.

All peptides, including native sequences and conservative variant sequences derived from peptides that bind the 15A.2 monoclonal antibody preferably contain at least one cysteine-two peptide bonds from a proline-histidine pair, which is three spaces from a cysteine, or contain the sequence cysteine-one peptide bond from a proline-histidine pair, one peptide bond from a praline, which is two peptide bonds from a cysteine, i.e., contain a sequence in the form of: SEQ ID NO:1 C-blank-blank-P-H-blank-blank-blank-C, or SEQ ID NO:2 C-blank-P-H-Blank-P-blank-blank-C. Blank can be any amino acid.

A host is actively immunized against allergic manifestations by administration of a peptide-containing molecule that is able to induce the formation of antibody capable of binding circulating IgE and/or down regulating the synthesis of endogenous IgE. This reduction in the serum level of IgE reduces, or eliminates, allergic responses in these animals.

Similarly, in accordance with the present invention, a specific binding molecule, such as monoclonal antibody 15A.2 or 8H.8, provides an anti-IgE moiety in dogs. This antibody, either alone or in combination, is used as a prophylactic agent for allergy, or is used therapeutically for dogs experiencing allergic disease, and effectively reduces or eliminates IgE-mediated allergic disease.

### T Cell Dependent Generation of Antibodies

As discussed below, there are mechanisms to induce antibodies that are independent of a need for T cell interactions. However, for T cell dependent mechanisms, if the patient's IgE antibody, recognized by its own B cell, should hold a foreign antigen in its binding site, then this foreign antigen will be internalized and processed in the same way that the IgE is internalized and processed. Peptides derived from this processed foreign antigen, in association with MHC, will therefore be presented on the surface of the B cell. Accordingly, a B cell reactive to a self-molecule will now also be presenting epitopes that are recognized by T cells initially responsible for mounting an immune response to a foreign antigen. These T cells will now help this auto-reactive B cell to produce auto-reactive antibodies.

To induce T cell dependent activation of B cells, generally a peptide immunogen, possibly a hapten, which defines an epitope recognized by a B cell, is conjugated to a carrier protein. For example, the epitope recognized by the B cell can be an epitope on surface-bound IgE. Typically the carrier protein is KLH or BSA. A large carrier protein, such as KLH, provides T cell epitopes required for the generation of an immune response directed against the hapten. The carrier protein contains peptide epitope sequences that activate T cells; in turn, these activated T cells help to generate an immune response to the conjugated peptide. An alternative mechanism for inducing T cell dependent activation of B cells is to prepare a recombinant fusion protein having the epitopes recognized by the B cell, e.g. self IgE epitopes, translated along with epitopes bound by T cells.

A unique approach to generate peptides via T cell dependent responses is contained in EPICOAT™ technology from Axis Genetics of Cambridge, England. Thus, in accordance with an embodiment of the invention, antibody epitopes, or conservative variants thereof, are inserted into the coat proteins of a cow pea virus. The virus is thus able to express these peptides on its surfaces, and present them to the immune system in order to induce antibody production. A perceived advantage of this embodiment is that the plant virus proteins are recognized as foreign by the immunized animal. The availability of epitopes on the virus particles bound by T cells induces a T cell dependent antibody response against the inserted self-peptides of interest. Thus, in accordance with the invention both B and T cell responses to the recombinant virus particle containing IgE epitopes from a given species are generated. Accordingly, a particularly effective mechanism for achieving auto-immunity to IgE is provided; this autoimmunity serves to lessen or eliminate allergic symptoms.

### T Cell Independent Generation of Antibodies

Multiply antigenic peptides (MAPs) were synthesized which contained four copies of the 11 N-terminal amino acids of canine IgE exon 3, i.e., a 4-MAP peptide. In dogs immunized with the 4-MAP peptide comprising the 11 amino acid N-terminal region of exon 3 discussed herein, a T-cell independent induction of an immune response is believed to occur.

Each 4-MAP peptide contains four identical peptide sequences of interest. This structural configuration provides for the possibility that B cells that recognize this structure may become activated in a T cell-independent fashion. An individual 4-MAP peptide has the ability to bind to multiple receptors on a memory B cell, and thereby to cross-link the receptors, whereupon signals that lead to the activation, maturation and elaboration of soluble auto-anti-IgE molecules from the B cell are induced without the need for T cell interactions.

In a traditional protein carrier/hapten immunogen system, the large carrier protein, such as KLH, provides T cell epitopes required for the generation of an immune response directed against the hapten. When using either MAP peptides, or an MLA adjuvant, these T cell epitopes are not present. Thus, the resulting immune response is independent of T cells.

### EXAMPLES

### Example 1

Results of the studies disclosed in this Example indicated that peptides derived from the heavy chain of canine IgE can be used as vaccines to generate an auto anti-IgE response that down-regulates the synthesis and/or decreases the half life of circulating IgE in dogs.

Four normal dogs were studied. Two dogs received a 4MAP peptide comprising four copies of the peptide E3a.5 which had been identified by epitope mapping experiments and phage display panning of the anti-canine IgE antibody 8H.8. The remaining two dogs were immunized with a recombinant protein corresponding to canine IgE exon 3a having the 71 C-terminal amino acids of exon 3 in a Pmal fusion protein designated PmalCex3a. The maltose binding protein, Pmal, was added in order to facilitate purification; in effect the Pmal protein functions like a carrier in a typical hapten-carrier system. This protein was prepared using the New England BioLabs Protein Fusion and Transfection System™. Additionally, each immunogen was formulated with either RIBI (Hamilton, MT) or VRI (Boston, MA) adjuvant as indicated. The two adjuvants were studied to see if either were particularly preferable as shown in Table 2.

**TABLE 2**

| | | **Initial IGE concentration** |
|---|---|---|
| Dog #1 | PmalCex3a + VRI adjuvant | 5.5 µg/mL |
| Dog #2 | PmalCex3a + RIBI adjuvant | 8 µg/mL |
| Dog #3 | E3a.5 + VRI adjuvant | 10 µg/mL |
| Dog #4 | E3a.5 + RIBI adjuvant | 22 µg/mL |

Animals were immunized IM (paw) with 100 µg of each antigen formulated with 500 µg of each adjuvant, in a final volume of 500 µl. Each animal was bled at three weeks post immunization, after which it received a boost immunization. A second bleed was obtained three weeks after this boost. A subsequent bleed was taken several weeks later. More specifically, the dogs were split into pairs and placed on separate immunization schedules as follows in Table 3:

**TABLE 3**

| | **pre- bleed** | **immunized** | **1**^{**st**} **bleed/boost** (Weeks Post-immunization) | **2**^{**nd**} **bleed** (Weeks Post-Immunization) | **3**^{**rd**} **bleed** (Weeks Post-Immunization) |
|---|---|---|---|---|---|
| Dogs 1&2 (exon 3a) | 8.7.97 | 8.26.97 | 9.16.97 (3 weeks) | 10.8.97 (6 weeks) | 10.24.97 (8 weeks) |
| Dogs 3&4 (peptide E3a.5) | 8.7.97 | 8.14.97 | 9.4.97 (3 weeks) | 9.25.97 (6 weeks) | 10.24.97 (10 weeks) |

Each serum sample was assayed for IgE concentration as well as auto anti-IgE activity.

**Table 4**

| **IgE Concentration of Immunized Dogs (µg/mL)** | | | | |
|---|---|---|---|---|
| | pre-bleed | 1^{st} Bleed | 2^{nd} Bleed | 3^{rd} Bleed |
| Dog #1 pmalCex3a + VRI | 6.92 | 3.41 | 3.91 | 2.43 |
| Dog #2 pmalCex3a + RIBI | 10.68 | 7.19 | 3.83 | 2.48 |
| Dog #3 E3a.5 + VRI | 10.43 | 3.57 | 6.51 | 3.94 |
| Dog #4 E3a.5 + RIBI | 25.68 | 7.65 | 9.27 | 4.67 |

**Table 5**

| **Percent Reduction of Serum IgE** | | | | |
|---|---|---|---|---|
| | pre-bleed | 1^{st} Bleed | 2^{nd} Bleed | 3^{rd} Bleed |
| Dog #1 pmalCex3a + VRI | N/A | 50% | 43% | 65% |
| Dog #2 pmalCex3a + RIBI | N/A | 33% | 64% | 77% |
| Dog #3 E3a.5 + VRI | N/A | 66% | 37% | 62% |
| Dog #4 E3a.5 + RIBI | N/A | 71% | 64% | 82% |

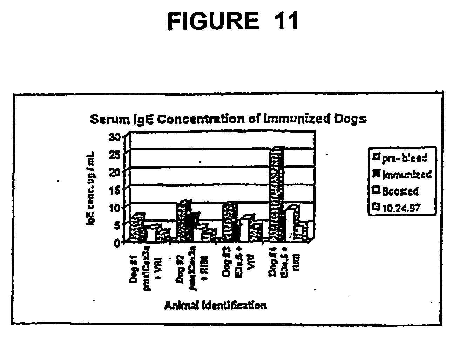

IgE concentration was measured using the Quik IgE ELISA assay. Tables 3 and 4 show results obtained from a single assay. Figure 11 graphically depicts these same results. IgE levels decreased from 50% to 71% at the time of the first bleed, which was 3 weeks post immunization ("immunized" data). Three weeks later, 3 of 4 dogs showed a slight rebound in IgE concentration, (although still depressed from pre-bleed levels), while the serum IgE of dog #2 continued to drop ("boosted" data). The IgE concentration in all experimental animals at the time of the final bleed was significantly lower than the pre-bleed and ranged from 65%; to 82% ("10.24.97" data). The results indicate that every immunization protocol resulted in decreased serum IgE concentration. It appears that the 3a.5 peptide was equally efficacious as the full length exon 3 recombinant peptide, and that RIBI adjuvant was somewhat better than VRI adjuvant in reducing serum IgE concentration.

It is to be noted that the 8H.8 antibody was initially raised to the exon 3a peptide; thus, the epitope bound by 8H.8 is available on the exon 3a peptide. Accordingly, administration of the exon 3a peptide in the Pmal fusion protein also served to administer the peptide sequence of the peptide E3a.5 in the Pmal form. Thus, the more significant reduction in IgE levels for dogs 3 and 4 appears to be correlated to the multiple antigenic peptide format in which the E3a.5 peptide was administered.

Moreover, auto anti-IgE antibody concentration was determined by ELISA. Purified canine IgE was coated on ELISA plates and the serum from the immunized animals was incubated on the solid phase overnight. Auto anti-IgE antibodies were detected using an horseradish peroxidase (HRPO) labeled anticanine IgG antibody designated 14A.3. Auto anti-IgE levels were not quantitated, but absorbance values were compared between pre-bleed samples and serum obtained from immunized animals.

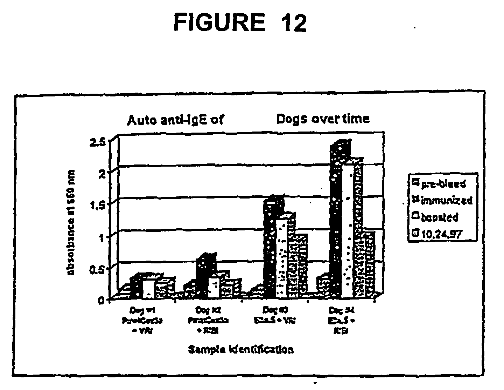

Figure 12 presents absorbance values representing auto anti-IgE antibody concentration in the experimental animals. Every animal exhibited an increase in anti-IgE antibodies followed by decreasing concentrations in subsequent bleeds.

Clearly the most pronounced auto anti-IgE response was detected in dogs 3 & 4. These animals had received the peptide immunogen E3a.5 as opposed to full length recombinant exon 3. Additionally, it also appeared that RIBI adjuvant resulted in a greater response than the VRI adjuvant. The decrease in auto anti-IgE activity over time may reflect the decrease in serum IgE shown above. Fewer anti-IgE antibodies may be produced as IgE is cleared from the serum of these animals.

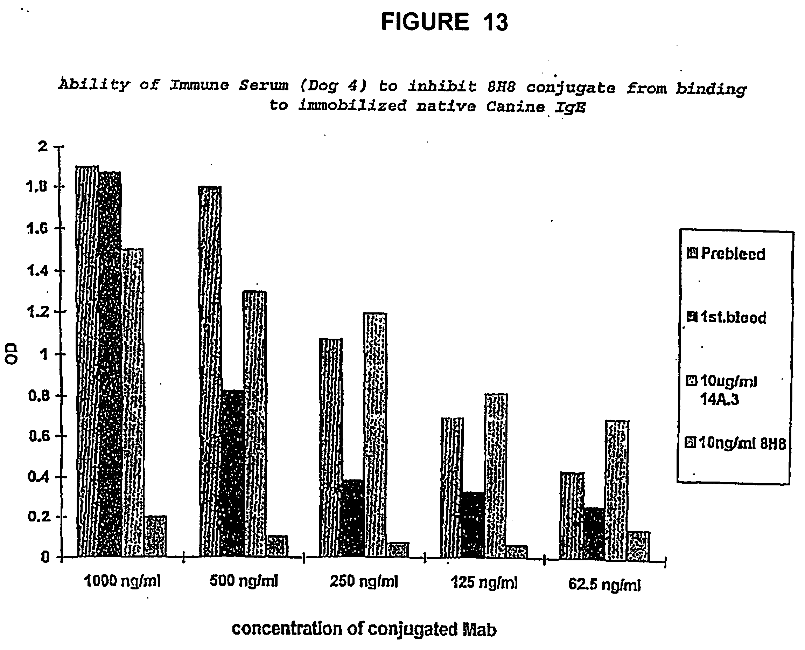

Additionally, as depicted in Figure 13, serum from an immunized dog was evaluated to see if it inhibited the binding of conjugated 8H.8 to Immulon 4 ELISA plates (Dynatech, Chantilly, VA) having affinity purified native IgE was immobilized thereon at 2 µg/ml, after which the plates were blocked with BSA. Individual wells, in duplicate, were pre-incubated with a 1/100 dilution in PBS of preimmunization (prebleed) and 3-week post-immunization (1st bleed) serum from Dog 4 which had been immunized and boosted with the E3a.5 4-map peptide with RIBI adjuvant. Positive and negative controls consisted of wells pre-incubated with 10µg,1 monoclonal antibody 14A.3 (neg. control) or 10 ng/m1 monoclonal antibody 8H.8 (positive control). Monoclonal antibody 14A.3 is known to recognize canine IgG. The wells were washed and a serial, 2-fold dilution of HRPO-conjugated monoclonal antibody 8H.8 starting at µg/m1 was added to each pre-incubated well. After one hour the plates were washed and a standard ELISA performed.

Thus, the data in Figure 13 demonstrate that both immune serum ("first bleed") and monoclonal antibody 8H.8 were able to block the binding of conjugated 8H.8 to immobilized canine IgE.

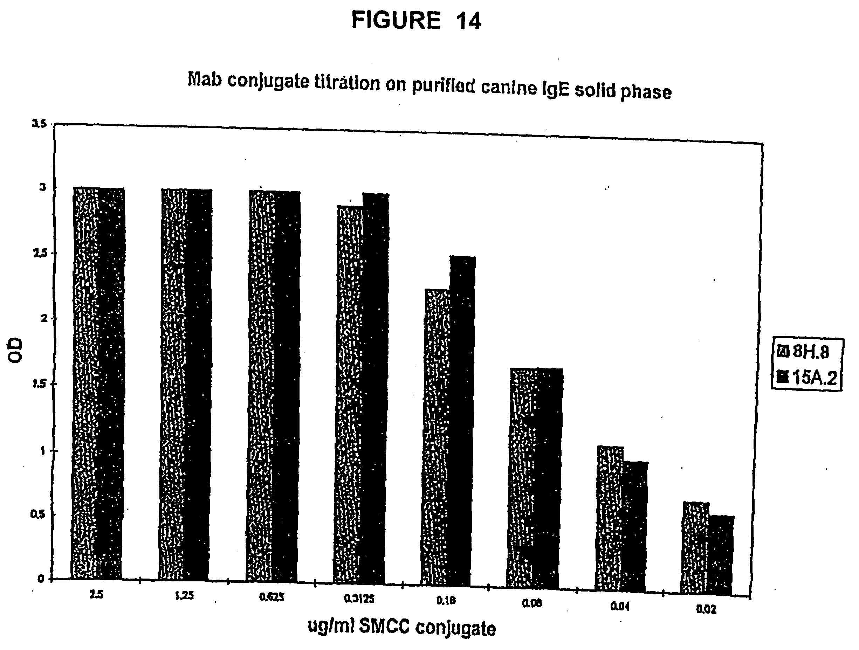

Figure 14 depicts results of the binding study where antibody 8H.8 and antibody 15A.2 were separately assayed to determine their ability to bind to a solid phase having affinity purified canine IgE immobilized thereon. These results showed that those 8H.8 and 15A.2 bind to native IgE when immobilized on a solid phase. The results from these studies suggest that the binding of each of these antibodies to the surface bound IgE was of comparable affinity. To determine whether the affinity of binding of each antibody was, in fact, comparable, further studies were performed.

Figure 15 depicts results of the study where antibody 8H.8 conjugated to a signal moiety was reacted at various concentration with solid phases having various peptides immobilized thereon. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon, data depicted by an (X) reflects data for a solid phase having an E3a.5 scrambled peptide immobilized thereon, data depicted by (*) depicts data for solid phase having the seven amino acid peptide identified by phage display technology to which SH8 binds immobilized thereon. Thus, it is seen that 8H.8 binds to each solid phase with the exception solid phase having the E3a.5 scrambled peptide.

Monoclonal antibody 14K2 is known to bind exclusive to canine IgE exon 4. This monoclonal antibody was reacted with various solid phases having different peptides immobilized thereon. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon; data depicted by an (X) reflects data for a solid phase having an E3a.5 scrambled peptide immobilized thereon; and data depicted by (*) depicts data for solid phase having the seven amino acid peptide identified by phage display technology to which 8H.8 binds immobilized thereon. Thus, it is seen that 14K2 only binds to a solid phase having the entire canine IgE molecule immobilized thereon. This data is depicted in Figure 16.

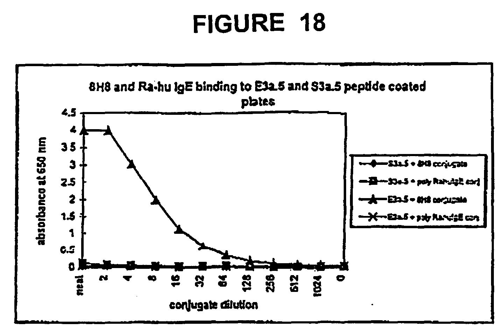

Monoclonal antibody 15A.2 is believed to bind only to canine IgE exon 3. Figure 17 depicts data for studies which evaluated the binding of 15A.2 to various peptides. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon, data depicted by an (X) reflects data for a solid phase having an E3a.5 scrambled peptide immobilized thereon, data depicted by (*) depicts data for solid phase having the seven amino acid peptide identified by phage display technology to which 8H.8 binds immobilized thereon. These binding studies showed that 15A.2 bound only to a solid phase which had the entire canine IgE molecule immobilized thereon. This data indicated that 15A.2 does not bind the same epitope as that bound by 8H.8.

Figure 18 depicts data for studies that compare the binding of 8H.8 or polyclonal antibodies raised to recombinant human IgE, to peptide E3a.5 or to peptide E3a.5 with an amino acid substitution making the peptide more analogous to a sequence in the human genome which encodes human IgE, designated peptide S3a.5. In Figure 18 data depicted by the (◆) reflects substituted 3a.5 compared with 8H.8; data depicted by (■) reflects substituted peptide 3a.5 and Re-Hu IgE: data depicted by (π) reflects peptide E3a.5 and 8H.8 and, data depicted by and (X) reflects data regarding peptide E3a.5 and polyclonal Re Hu IgE. These data indicated that only 8H.8 became bound to E3a.5 on a solid phase, no other combination exhibited binding.

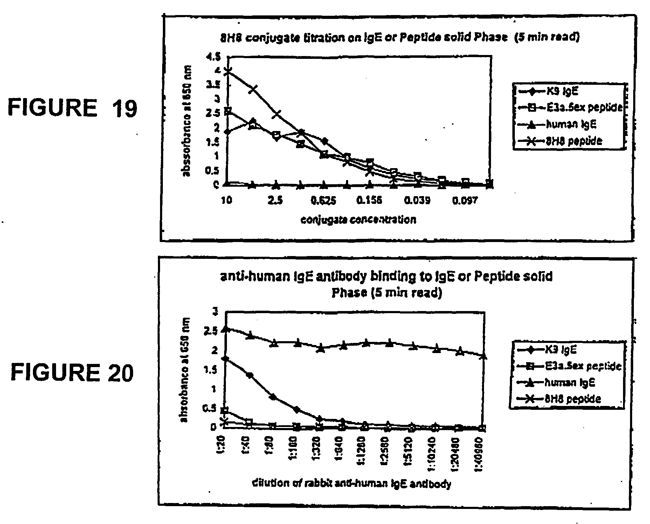

Figure 19 depicts the data on studies that compared the binding of 8H.8 to various peptides immobilized on a solid phase. Data depicted by (◆) reflects data for a solid phase having canine IgE immobilized thereon; data depicted by (■) reflects data for a solid phase having a peptide E3a.5 extended immobilized thereon; data depicted by (π) reflects data for a solid phase having human IgE immobilized thereon and, data depicted by an (X) reflects data for a solid phase having a 8H.8 peptide immobilized thereon. Accordingly it is seen that 8H.8 binds to canine IgE, peptide E3a.5 extended or the seven amino acid peptide identified by phage display technology as the binding epitope for 8H.8, when each of these peptides were immobilized on a solid phase. Additionally, it is seen that 8H.8 does not bind to human IgE.

Figure 20 depicts data for studies which compare the ability of polyclonal anti human IgE antibodies ability to bind to solid phases having various peptides immobilized thereon. Data depicted by (◆) reflects data for a solid phase having canine IgE immobilized thereon; data depicted by (■) reflects data for a solid phaae having a peptide E3a.5 extended immobilized thereon; data depicted by (π) reflects data for a solid phase having human IgE immobilized thereon and, data depicted by an (X) reflects data for a solid phase having a 8H.8 peptide immobilized thereon. Thus, it was seen that the anti-human IgE polyclonal antibodies bound to human IgE when immobilized on a solid phase. It was also seen that the polyclonal antiserum exhibited limited binding to peptide E3a.5 extended when the polyclonal antibodies were present at very high concentrations; this was believed to be a binding artifact due to the high antibody concentration. Thus, it was found that antibodies to Human IgE do not specifically bind to canine *I*gE or to peptides in accordance with the invention.

### Example 2

This Example repeats the study of Example 1, along with additional control substances. Thus, the 4-MAP E3a.5 peptide formulation used in Example 1 is used along with derivatives of this peptide. Two peptides (E3a.5 scrambled, and TM#2 FIV) serve as the negative controls. This example also evaluates an extended peptide from the C-terminal region from exon 3 of the IgE heavy chain for an ability to generate an auto anti-IgE response. All peptides are administered to the dog in a 4-MAP configuration.

Thus, the following peptides are used as set forth in Table 6:

**Table 6**

| **Peptide name** | **Sequence** | **Number of Amino Acids** | **SEQ ID NO.** |
|---|---|---|---|
| E3a.5 | GMNLTWYRESK | 11 | 10 |
| E3A.5 Extended | GMNLTWYRESEPVNPVPLLNKK | 23 | 12 |
| E3A.5-Scrambled | TYSKNMWERGL | 11 | 13 |
| TM#2 FIV | NQNQFFCKIPP | 11 | 14 |

Dogs immunized with these peptides are monitored for the presence of auto anti-IgE antibodies and circulating IgE concentration. Additionally, receptor-bound IgE on mast cells is evaluated by measuring the skin test response of these animals with crosslinking antibodies.

Accordingly, upon administration of peptide E3a.5 and peptide E3a.5 extended, canine auto anti-IgE antibodies are induced; these antibodies led to a decrease in circulating IgE levels. Skin test responses following administration of crosslinking antibodies show decreased levels of IgE on mast cells in the dogs which received peptides E3a.5 and E3a.5 extended. Thus, skin test reactions (wheal and flare) will be reduced or eliminated in response to cross-linking monoclonal antibodies.

The dogs to which E3a.5 scrambled and TM#2 FIV are administered show no IgE response. However, the E3a.5 scrambled peptide induces an immune response specific for the peptide, but for the levels of IgE remain unchanged; a similar response is produced by the FIV peptide.

### Example 3

In this Example, the E3a.5 peptide was used in conjunction with the KLH carrier molecule in order to evaluate T cell dependent immune responses to self peptides derived from IgE. Appropriate negative controls for the canine peptide (e.g., TM#2 FIV), are also used.

In a traditional protein carrier/hapten immunogen system, a carrier protein, such as KLH, provides T cell epitopes required for the generation of an immune response directed against the hapten. Thus, in this example, KLH adjuvant serves to evaluate a classic T cell dependant immune response.

This example, therefore, evaluates T cell dependant immune response. In this example, four dogs are evaluated: two 2 dogs per immunogen, as follows:

**Table 7**

| **PEPTIDE** | **CARRIER SYSTEM** | **T CELL DEPENDENT OR INDEPENDENT** |
|---|---|---|
| E3a.5 | KLH | DEPENDENT |
| TM#2FIV | KLH | DEPENDENT |

Accordingly, upon administration of peptide E3a.5 with a KLH carrier, canine auto anti-IgE antibodies are induced; these antibodies led to a decrease in circulating IgE levels. Skin test responses following administration of crosslinking antibodies show decreased levels of IgE on mast cells in the dogs which received peptide E3a.5 with the KLH carrier. Thus, E3a.5-KLH molecule induces auto-anti IgE immunoglobulin production.

The dogs to which TM#2 FIV are administered show no IgE response. The FIV-KLH molecule induces antibody production specific for the FIV peptide, but produces no change in serum IgE levels.

### Example 4

In this Example, the E3a.5 peptide was used in 10conjunction with a carrier molecule in order to evaluate T cell independent immune responses to self peptides derived from IgE. Appropriate negative controls for the E3a.5 peptide (e.g., TM#2 FIV), are also used.

In a traditional protein carrier/hapten immunogen system, a carrier protein, such as KLH, provides T cell epitopes required for the generation of an immune response directed against the hapten. However, when using either MAP peptides, or the MLA adjuvant, these epitopes are not present. Thus, any resulting immune response is independent of helper T cells.

This example, therefore, evaluates T cell independent immune response. In this example, four dogs are evaluated: two 2 Dogs per immunogen, as follows:

**Table 8**

| **PEPTIDE** | **CARRIER SYSTEM** | **T CELL DEPENDENT OR INDEPENDENT** |
|---|---|---|
| E3a.5 | MLA | INDEPENDENT |
| TM#2FIV | MLA | INDEPENDENT |

Accordingly, upon administration of peptide E3a.5 with an MLA carrier, canine auto anti-IgE antibodies are induced in a T cell independent manner; these antibodies led to a decrease in circulating IgE levels. Skin test responses following administration of crosslinking antibodies show decreased levels of IgE on mast cells in the dogs which received peptide E3a.5 with the MLA carrier. The dogs to which TM#2 FIV in MLA carrier are administered show no response. Thus, E3a.5-MLA induces auto-anti IgE immunoglobulin production.

The dogs to which TM#2 FIV are administered show no IgE response. The FIV-MLA molecule induces antibody production specific for the FIV peptide, but produces no change in serum IgE levels.

### Example 5

In this Example, the E3a.5 peptide was used in conjunction with a carrier molecule derived from a plant virus protein, in order to evaluate T cell dependent immune responses to self peptides derived from IgE. Appropriate negative controls for the E3a.5 peptide (e.g., TM#2 FIV), are also used in the same plant virus particle format.

In a traditional protein carrier/hapten immunogen system, a carrier protein, such as KLH, provides T cell epitopes required for the generation of an immune response directed against the hapten. The present approach for generating antibodies to peptide antigens involves placing multiple copies of a peptide in accordance with the invention on the surface of plant virus particles. EPICOAT™ technology (Axis Genetics p1c, Cambridge, England) is the presently preferred system for producing such particles. The EPICOAT™ technology is based on chimeric virus particle (CVP technology which utilizes the recombinant genetic modification of plant viruses.

The EPICOAT™ technology is currently based on the cow pea mosaic virus (CPMV) a plant virus that infects the cow pea plant, also known as the "black-eyed" bean. The CPMV particle is icosahedral, and about twenty-eight nanometers (nm) in diameter. CPMV particles are composed of two proteins, referred to as the large and small coat proteins. Studies have revealed a site within the small coat protein which allows presentation of a foreign peptide, i.e., a peptide in accordance with the invention, in a prominent position on the virus surface, whereby up to sixty copies of the peptide are presented on each virus particle.

Thus, with the EPICOAT™ technology, DNA copies of the plant virus's genetic material are used. A minute quantity of the DNA encoding the virus protein, including the inserted foreign peptide, is applied to the leaves of young cow pea plant, along with an abrasive powder. The leaves are gently rubbed, and DNA enters the leaves and utilizes the plant's own cellular mechanisms to initiate generation of functional virus particles. The virus replicates within the inoculated leaves and spreads throughout the growing plant.

After two to three weeks, leaf material containing large quantities of the virus is harvested. Chimeric virus particles (CVPs) are isolated by centrifugation and selective precipitation of homogenized plant material. Between 1 to 2 grams of CVPs are obtained per kilogram fresh weight of leaf material. Advantageously, cow pea plants are readily grown in abundance in controlled environments, allowing generation of large quantities of CVPs containing many copies of peptides in accordance with the invention.

Accordingly, with the EPICOAT™ technology, separate studies are preformed where the following peptides in accordance with the invention are placed on plant particles: E3a.5 peptide, exon 3a peptide; E3a.5 extended peptide, 8H.8 mimotope peptide (7 amino acids), and TM#2FIV peptide as control. The peptides are inserted into the plant virus in such a way that multiple copies of the peptide are displayed on the surface of the virus particle.

This example, therefore, evaluates T cell dependent immune response. The T cell binding epitopes are provided by the viral proteins, and it is believed that these viral proteins are potent inducers of T cell dependent immunological mechanisms. In this example, ten dogs are evaluated: two dogs per immunogen, as follows:

**Table 9**

| **PEPTIDE** | **CARRIER SYSTEM** | **T CELL DEPENDENT OR INDEPENDENT** |
|---|---|---|
| E3a.5 | chimeric virus particle | DEPENDENT |
| E3a.5 extended | chimeric virus particle | DEPENDENT |
| exon 3a | chimeric virus particle | DEPENDENT |
| 8H.8 mimotope | chimeric virus particle | DEPENDENT |
| TM#2FIV | chimeric virus particle | DEPENDENT |

Accordingly, upon administration of peptide E3a.5 with an chimeric virus particle carrier (CVP), canine auto anti-IgE antibodies are induced in a T cell dependent manner for CVPs which contain each of the following peptides: E3a.5, E3a.5 extended, exon 3a, and, 8H.8 mimotope. These antibodies led to a decrease in circulating IgE levels. Skin test responses following administration of crosslinking antibodies show decreased levels of IgE on mast cells in the dogs which received any of peptides E3a.5, E3a.5 extended, exon 3a, and, 8H.8 mimotope in a CVP carrier form. Thus, E3a.5-CVP, E3a.5 extended-CVP, exon 3a-CVP, and, 8H.8 mimotope-CVP each induce auto-anti IgE immunoglobulin production.

In contrast, the dogs to which TM#2 FIV in CVP carrier are administered show no IgE response. The FIV-CVP molecule induces antibody production specific for the FIV peptide, but produces no change in serum IgE levels.

### Example 6

The amino acid sequences for the epitope bound by 8H.8, the 11 amino acid sequence used in the immunization studies presented herein, as well as analogous sequences from feline and Human IgE sequences are set forth in Table 10. Additionally, a substituted canine sequence was prepared having an amino acid substitution which made the peptide more closely analogous to the human peptide.

**TABLE 10**

| **Species/Source** | **Sequence** | **SEQ ID NO.** |
|---|---|---|
| mimotope bound by 8H.8 | T-L-L-E-Y-R-M | 9 |
| canine | G-M-N-L-T-W-Y-R-E-S-K | 10 |
| human | V-N-L-T-W-S-R | 15 |
| feline | G-M-T-L-T-W-S-R-E-N-G | 16 |
| Substituted Canine Sequence | G-M-N-L-T-W-S-R-E-S-K | 17 |

Each of the peptides in Table 10 was placed on a solid phase and studies were performed to identify if 8H.8 would bind to the surface-bound peptide. It was found that 8H.8 bound to a surface having the mimotope peptide thereon, and to a surface having the 11 amino acid peptide bound thereon. It was found that 8H.8 did not bind to the human peptide, the feline peptide or to the canine sequence which had an amino acid substitution which made it more analogous to the human sequence, when either of these peptides were surface bound.

### Closing

As used herein and in the appended claims, the singular forms "a," "and," and "the" include plural referents unless the context clearly dictates otherwise. Thus, for example, reference to "a formulation" includes mixtures of different formulations and reference to "the method of treatment" includes reference to equivalent steps and methods known to those skilled in the art, and so forth.

Unless defined otherwise, all technical and scientific terms used herein have the same meaning as commonly understood by one of ordinary skill in the art to which this invention belongs. Although any methods and materials similar to or equivalent to those described herein can be used in the practice or testing of the invention, the preferred methods and materials are now described. All publications mentioned herein are fully incorporated herein by reference.

## Claims

1. An isolated and purified peptide comprising a leucine positioned two peptide bonds away from a tyrosinearginine pair.

2. The peptide of claim 1 comprising the form: leucine-blank-blank-tyrosine-arginine, tyrosine-arginine-blank-blank-leucine, or, leucine-blank-blank-tyrosine-arginine-blank-blank-leucine, where blank is any amino acid.

3. The peptide of claim 2 wherein at least one blank is an amino acid with an aromatic ring.

4. The peptide of claim 1 comprising from 5 to 71 amino acids.

5. The peptide of claim 4 further comprising an amino acid sequence, or a conservative variant thereof, which comprises: Thr-Leu-Leu-Glu-Tyr-Arg-Met or which comprises: Gly-Met-Asn-Leu-Thr-Trp-Tyr-Arg-Glu-Ser-Lys.

6. An isolated and purified peptide comprising cysteine-blank-blank-proline-histidine-blank-blank-blank-cysteine, where blank is any amino acid.

7. The peptide of claim 6 comprising cysteine-proline-asparagine-proline-histidine-isoleucine-proline-methionine-cysteine or cysteine-proline-asparagine-proline-histidine-asparagine-proline-tyrosine-cysteine.

8. The peptide of claim 7 comprising serine-valine-threonine-leucine-cysteine-proline-asparagine-proline-histicline-isoleucine-proline-methionine-cysteine-glycine-glycine-glycine or serine-alanine-cysteine-proline-asparagine-proline-histidine-asparagine-proline-tyrosine-cysteine-glycine-glycine-glycine.

9. The peptide of claim 6 comprising from 5 to 71 amino acids.

10. An isolated and purified peptide comprising cysteine-blank-proline-histidine-blank-proline-blank-blank-cysteine, where blank is any amino acid.

11. The peptide of claim 10 comprising cysteine-histidine-proline-histidine-leucine-proline-lysine-serine-cysteine.

12. The peptide of claim 11 comprising serine-alanine-cysteine-histidine-proline-histidine-leucine-proline-lysine-serine-cysteine-glycine-glycine-glycine.

13. The peptide of claim 10 comprising from 5 to 71 amino acids.

14. A conservative variant of a peptide of claim 1, 6 or 10.

15. A multiply antigenic peptide comprising multiple copies of a peptide of claim 1, 6 or 10 or multiple copies of a conservative variant of a peptide of claim 1, 6 or 10.

16. A recombinant plant virus particle comprising a peptide of claim 1, 6 or 10 or a conservative variant thereof.

17. The use of a peptide of claim 1, 6 or 10 for the manufacture of a pharmaceutical composition for generating canine autoantibodies directed to the canine IgE molecule.

18. The use of claim 17, wherein the pharmaceutical composition further comprises an adjuvant.

19. The use of claim 18, wherein the pharmaceutical composition comprises the adjuvant at a weight ratio of one part peptide to five parts adjuvant.

20. The use of claim 18 for the manufacture of a pharmaceutical composition kit, said kit comprising a first dose of said peptide and a booster dose of said peptide.

21. The use of a peptide of claim 1, 6 or 10 for the manufacture of a pharmaceutical composition for treatment or prophylaxis of allergic symptoms in a dog.

22. An immunogenic molecule comprising a peptide of claim 1, 6 or 10, or a conservative variant thereof, linked to a carrier molecule capable of facilitating an immune response.

23. The immunogenic molecule of claim 22 wherein the carrier molecule is KLH, BSA, MLA, a multiply antigenic peptide, or a plant virus particle.

24. An antibody which specifically binds to a peptide of claim 1, 6 or 10 or a conservative variant thereof.

25. An antibody of claim 24 that is raised to the peptide or variant.
